# EUROPÄISCHE PATENTSCHRIFT

(11) **EP 0 865 438 B1**
(45) Veröffentlichungstag und Bekanntmachung des Hinweises auf die Patenterteilung: **17.10.2001**
(21) Anmeldenummer: 96939080.6
(22) Anmeldetag: 18.11.1996
(51) Int. Cl.: C07D 309/36, C07D 311/74, C07D 409/04, C07D 405/04, C07D 407/04, A01N 43/16

(54) **OXYMETHOXY-3-ARYL-PYRON-DERIVATE**
OXYMETHOXY-3-ARYL-PYRONE DERIVATIVES
DERIVES D'OXYMETHOXY-3-ARYL-PYRONE

(30) Priorität: 29.11.1995 DE 19544457
(43) Veröffentlichungstag der Anmeldung: 23.09.1998
(73) Patentinhaber: BAYER AG, 51368 Leverkusen (DE)
(72) Erfinder: BRETSCHNEIDER, Thomas, D-53797 Lohmar (DE); FISCHER, Reiner, D-40789 Monheim (DE); LIEB, Folker, D-51375 Leverkusen (DE); HAGEMANN, Hermann, D-51375 Leverkusen (DE); RUTHER, Michael, D-40789 Monheim (DE); STETTER, Jörg, D-42115 Wuppertal (DE); ANDERSCH, Wolfram, D-51469 Bergisch Gladbach (DE); ERDELEN, Christoph, D-42799 Leichlingen (DE); HÄNSSLER, Gerd, D-51381 Leverkusen (DE); MENCKE, Norbert, D-51381 Leverkusen (DE); STENZEL, Klaus, D-40595 Düsseldorf (DE); TURBERG, Andreas, D-40699 Erkrath (DE); WACHENDORFF-NEUMANN, Ulrike, D-56566 Neuwied (DE)
(86) Internationale Anmeldenummer: EP9605058
(87) Internationale Veröffentlichungsnummer: WO9719941

(56) Entgegenhaltungen:
- EP-A- 0 588 137

## Beschreibung

Die Erfindung betrifft neue Oxymethoxy-3-aryl-pyron-Derivate, ein Verfahren zu ihrer Herstellung und ihre Verwendung als Schädlingsbekämpfungsmittel, Fungizide und Herbizide.

Bestimmte, im Phenylring unsubstituierte Phenyl-pyron-Derivate sind bereits bekannt geworden (vgl. A.M. Chirazi, T. Kappe und E. Ziegler, Arch. Pharm. 309, 558 (1976) und K.-H. Boltze und K. Heidenbluth, Chem. Ber. 91, 2849 (1958)), wobei für diese Verbindungen eine mögliche Verwendbarkeit als Schädlingsbekämpfungsmittel nicht angegeben wird. Im Phenylring substituierte Phenyl-pyron-Derivate mit herbiziden, akariziden und insektiziden Eigenschaften sind in EP-A-588 137 beschrieben.

Die Wirksamkeit und Wirkungsbreite dieser Verbindungen ist jedoch insbesondere bei niedrigen Aufwandmengen und Konzentrationen nicht immer voll zufriedenstellend. Daneben ist die Pflanzenverträglichkeit häufig nicht ausreichend.

### Es wurden nun neue Verbindungen der Formel (I)

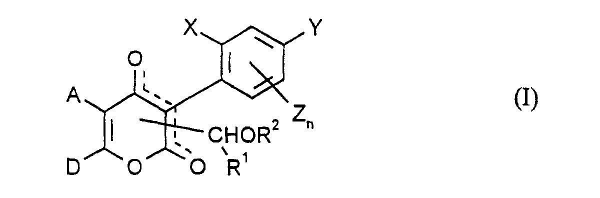

gefunden,
in welcher
- X: für Halogen, Nitro, Cyano, Alkyl, Alkenyl, Alkoxy, Alkenyloxy, Alkylthio, Alkylsulfinyl, Alkylsulfonyl, Halogenalkyl, Halogenalkenyl, Halogenalkoxy, Halogenalkenyloxy oder jeweils gegebenenfalls substituiertes Phenyl, Phenoxy, Phenylthio, Benzyloxy oder Benzylthio steht,
- Y: für Wasserstoff, Halogen, Nitro, Cyano, Alkyl, Alkenyl, Alkoxy, Alkenyloxy, Alkylthio, Alkylsulfinyl, Alkylsulfonyl, Halogenalkyl, Halogenalkenyl, Halogenalkoxy oder Halogenalkenyloxy steht,
- Z: für Halogen, Nitro, Cyano, Alkyl, Alkenyl, Alkoxy, Alkenyloxy, Halogenalkyl, Halogenalkenyl, Halogenalkoxy oder Halogenalkenyloxy steht,
- n: für eine der Zahlen 0, 1, 2 oder 3 steht,
- A: für Wasserstoff, Halogen, für einen gegebenenfalls substituierten Rest aus der Reihe Alkyl, Cycloalkyl, Alkenyl, Alkinyl, Arylalkyl, Aryl, Hetarylalkyl oder Hetaryl oder für eine der Gruppen -COR³, -CO₂R³, -CN, -CONR³R⁴, -SO₂R³ oder -P(O)(OR³)OR⁴ steht, worin
R³ und R⁴ unabhängig voneinander für Wasserstoff oder für jeweils gegebenenfalls substituiertes Alkyl, Alkenyl, Arylalkyl, Aryl, Hetarylalkyl oder Hetaryl stehen oder
R³ und R⁴ gemeinsam für eine gegebenenfalls substituierte Alkylen-Gruppe stehen, in welcher gegebenenfalls eine oder mehrere Methylengruppen durch die gleiche Anzahl Heteroatome ersetzt ist,
- D: für Wasserstoff oder für einen gegebenenfalls substituierten Rest aus der Reihe Alkyl, Alkenyl, Alkinyl, Alkoxyalkyl, Polyalkoxyalkyl, Alkylthioalkyl, gesättigtes oder ungesättigtes Cycloalkyl, gesättigtes oder ungesättigtes Heterocyclyl, Arylalkyl, Aryl, Hetarylalkyl und Hetaryl steht oder
- A und D: gemeinsam für eine jeweils gegebenenfalls substituierte Alkylen- oder Alkenylen-Gruppe stehen, in welcher jeweils gegebenenfalls eine oder mehrere Methylengruppen durch die gleiche Anzahl Heteroatome oder Heterogruppen ersetzt ist,
- R¹: für Wasserstoff oder für gegebenenfalls durch Halogen substituiertes Alkyl steht und
- R²: für jeweils gegebenenfalls durch Halogen substituiertes Alkyl, Alkenyl oder Alkinyl steht.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Die Verbindungen der Formel (I) können in Abhängigkeit von der Stellung der Ketogruppe in den zwei isomeren Formen der Formeln (I)ₐ und (I)_{b}, vorliegen, 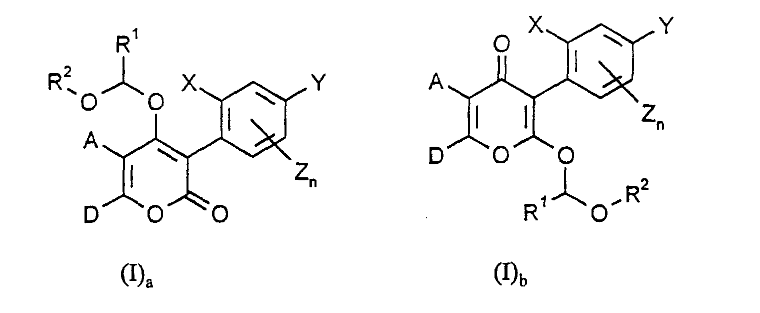 was durch die gestrichelte Linie in der Formel (I) zum Ausdruck gebracht werden soll.

Die Verbindungen der Formeln (I)ₐ und (I)_{b} können sowohl als Gemische als auch in Form ihrer reinen Isomeren vorliegen. Gemische der Verbindungen der Formeln (I)ₐ und (I)_{b} lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt ein, daß die Verbindungen gegebenenfalls in Form der Isomerengemische oder in der jeweils anderen isomeren Form vorliegen können.

### Weiterhin wurde gefunden, daß man die neuen Verbindungen der Formel (I)

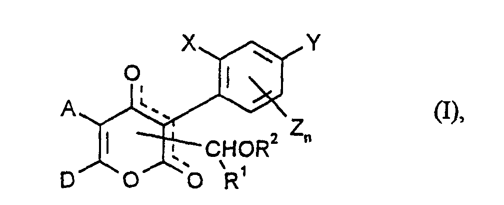

in welcher
- A, D, X, Y, Z, R¹, R² und n: die oben angegebenen Bedeutungen haben,

### erhält, wenn man Verbindungen der Formel (II)

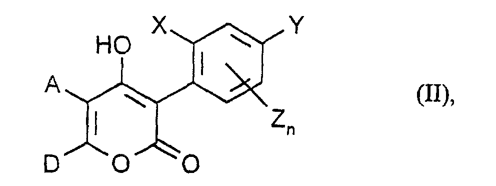

in welcher
- A, D, X, Y, Z und n: die oben angegebenen Bedeutungen haben, mit Verbindungen der Formel (III) 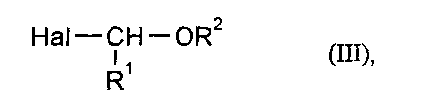
in welcher
- R¹ und R²: die oben angegebenen Bedeutungen haben und
- Hal: für Halogen (vorzugsweise für Chlor oder Brom) steht,
gegebenenfalls in Gegenwart eines Verdünnungsmittels und gegebenenfalls in Gegenwart eines Reaktionshilfsmittels umsetzt.

Weiterhin wurde gefunden, daß die neuen Verbindungen der Formel (I) bei guter Pflanzenverträglichkeit eine sehr gute Wirksamkeit als Schädlingsbekämpfungsmittel, vorzugsweise als Insektizide und Akarizide aber auch gegen Parasiten in der Nutztierhaltung aufweisen. Darüberhinaus weisen die neuen Verbindungen der Formel (I) z.T. sehr gute mikrobizide, vorzugsweise fungizide Wirkung auf. Herbizide Wirkungen wurden vorzugsweise bei höheren Aufwandmengen gefunden.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert:
- X: steht bevorzugt für Fluor, Chlor, Brom, Iod, Nitro, Cyano, C₁-C₈-Alkyl, C₂-C₆-Alkenyl, C₁-C₆-Alkoxy, C₂-C₆-Alkenyloxy, C₁-C₆-Alkylthio, C₁-C₆-Alkylsulfinyl, C₁-C₆-Alkylsulfonyl, für jeweils durch Fluor, Chlor oder Brom substituiertes C₁-C₆-Alkyl, C₂-C₆-Alkenyl, C₁-C₆-Alkoxy oder C₂-C₆-Alkenyloxy oder für jeweils gegebenenfalls durch Fluor, Chlor, Brom, Iod, Nitro, Cyano oder durch jeweils gegebenenfalls durch Fluor, Chlor oder Brom substituiertes C₁-C₆-Alkyl oder C₁-C₆-Alkoxy substituiertes Phenyl, Phenoxy, Phenylthio, Benzyloxy oder Benzylthio.
- Y: steht bevorzugt für Wasserstoff, Fluor, Chlor, Brom, Iod, Nitro, Cyano, C₁-C₈-Alkyl, C₂-C₆-Alkenyl, C₁-C₆-Alkoxy, C₂-C₆-Alkenyloxy, C₁-C₆-Al-kylthio, C₁-C₆-Alkylsulfinyl, C₁-C₆-Alkylsulfonyl oder für jeweils durch Fluor, Chlor oder Brom substituiertes C₁-C₆-Alkyl, C₂-C₆-Alkenyl, C₁-C₆-Alkoxy oder C₂-C₆-Alkenyloxy.
- Z: steht bevorzugt für Fluor, Chlor, Brom, Iod, Nitro, Cyano, C₁-C₈-Alkyl, C₂-C₆-Alkenyl, C₁-C₆-Alkoxy, C₂-C₆-Alkenyloxy oder jeweils durch Fluor, Chlor oder Brom substituiertes C₁-C₆-Alkyl, C₂-C₆-Alkenyl, C₁-C₆-Alkoxy oder C₂-C₆-Alkenyloxy.
- n: steht bevorzugt für eine der Zahlen 0, 1, 2 oder 3.
- A: steht bevorzugt für Wasserstoff, Fluor, Chlor, Brom oder Iod, für einen gegebenenfalls durch Fluor, Chlor, Brom oder Iod substituierten Rest aus der Reihe C₁-C₁₀-Alkyl, C₃-C₁₀-Cycloalkyl, C₃-C₁₀-Alkenyl und C₃-C₁₀-Alkinyl, für einen gegebenenfalls durch Fluor, Chlor, Brom, Iod, Nitro, C₁-C₈-Alkyl, C₁-C₈-Alkoxy, C₁-C₈-Alkylthio, C₁-C₈-Halogenalkyl, C₁-C₈-Halogenalkoxy oder Cyano substituierten Rest aus der Reihe Phenyl, Naphthyl, 5- oder 6-gliedriges Hetaryl mit ein bis drei Heteroatomen aus der Reihe Sauerstoff, Schwefel und Stickstoff, Phenyl-C₁-C₆-alkyl und 5-oder 6-gliedriges Hetaryl-C₁-C₆-alkyl mit ein bis drei Heteroatomen aus der Reihe Sauerstoff, Schwefel und Stickstoff oder für eine der Gruppen -COR³, -CO₂R³, -CN, -CONR³R⁴, -SO₂R³ oder -P(O)(OR³)OR⁴, worin
R³ und R⁴ unabhängig voneinander für Wasserstoff, für jeweils gegebenenfalls durch Fluor, Chlor, Brom oder Iod substituiertes C₁-C₁₀-Al-kyl oder C₃-C₁₀-Alkenyl oder für einen gegebenenfalls durch Fluor, Chlor, Brom, Iod, Nitro, C₁-C₈-Alkyl, C₁-C₈-Alkoxy, C₁-C₈-Alkylthio, C₁-C₈-Halogenalkyl, C₁-C₈-Halogenalkoxy oder Cyano substituierten Rest aus der Reihe Phenyl, Naphthyl, 5- oder 6-gliedriges Hetaryl mit ein bis drei Heteroatomen aus der Reihe Sauerstoff, Schwefel und Stickstoff, Phenyl-C₁-C₆-alkyl und 5- oder 6-gliedriges Hetaryl-C₁-C₆-alkyl mit ein bis drei Heteroatomen aus der Reihe Sauerstoff, Schwefel und Stickstoff stehen oder
R³ und R⁴ gemeinsam für eine C₂-C₇-Alkylen-Gruppe stehen, in welcher gegebenenfalls eine nicht endständige Methylengruppe durch Sauerstoff, Schwefel, NH oder N-C₁-C₄-Alkyl ersetzt ist.
- D: steht bevorzugt für Wasserstoff, für jeweils gegebenenfalls durch Fluor, Chlor, Brom oder Iod substituiertes C₁-C₁₂-Alkyl, C₃-C₈-Alkenyl, C₃-C₈-Alkinyl, C₁-C₁₀-Alkoxy-C₂-C₈-alkyl, Poly-C₁-C₈-alkoxy-C₂-C₈-alkyl oder C₁-C₁₀-Alkylthio-C₂-C₈-alkyl, für durch Cyano, C₁-C₈-Alkyloxycarbonyl oder C₁-C₈-Alkylcarbonyloxy substituiertes C₁-C₁₂-Alkyl, für gegebenenfalls durch Halogen, C₁-C₄-Alkyl, C₁-C₄-Alkoxy oder C₁-C₄-Halogenalkyl substituiertes C₃-C₈-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Hetaryl mit 5 bis 6 Ringatomen und ein bis drei Heteroatomen aus der Reihe Sauerstoff, Schwefel und Stickstoff, Phenyl-C₁-C₆-alkyl oder Hetaryl-C₁-C₆-alkyl mit 5 bis 6 Ringatomen und ein bis drei Heteroatomen aus der Reihe Sauerstoff, Schwefel und Stickstoff oder
- A und D: stehen gemeinsam bevorzugt für eine jeweils gegebenenfalls durch C₁-C₈-Alkyl, Fluor, Chlor, Brom oder Iod substituierte C₂-C₇-Alkylen- oder C₂-C₇-Alkenylen-Gruppe, worin gegebenenfalls zwei Kohlenstoffatome durch eine C₁-C₂-Alkylengruppe verbunden sind und in welcher gegebenenfalls eine Methylengruppe durch Sauerstoff, Schwefel, NH, N-C₁-C₄-Alkyl oder die Gruppe -O-CO- ersetzt ist.
- R¹: steht bevorzugt für Wasserstoff oder für gegebenenfalls durch Fluor, Chlor, Brom oder Iod substituiertes C₁-C₈-Alkyl.
- R²: steht bevorzugt für jeweils gegebenenfalls durch Fluor, Chlor, Brom oder Iod substituiertes C₁-C₁₀-Alkyl, C₃-C₁₀-Alkenyl oder C₃-C₁₀-Alkinyl.
- X: steht besonders bevorzugt für Fluor, Chlor, Brom, Nitro, Cyano, C₁-C₆-Alkyl, C₂-C₄-Alkenyl, C₁-C₄-Alkoxy, C₂-C₄-Alkenyloxy, C₁-C₄-Alkylthio, C₁-C₄-Alkylsulfinyl, C₁-C₄-Alkylsulfonyl, für jeweils durch Fluor oder Chlor substituiertes C₁-C₄-Alkyl, C₂-C₄-Alkenyl, C₁-C₄-Alkoxy oder C₂-C₄-Alkenyloxy oder für jeweils gegebenenfalls durch Fluor, Chlor, Brom, Nitro, Cyano oder durch jeweils gegebenenfalls durch Fluor oder Chlor substituiertes C₁-C₄-Alkyl oder C₁-C₄-Alkoxy substituiertes Phenyl, Phenoxy, Phenylthio, Benzyloxy oder Benzylthio.
- Y: steht besonders bevorzugt für Wasserstoff, Fluor, Chlor, Brom, Nitro, Cyano, C₁-C₆-Alkyl, C₂-C₄-Alkenyl, C₁-C₄-Alkoxy, C₂-C₄-Alkenyloxy, C₁-C₄-Alkylthio, C₁-C₄-Alkylsulfinyl, C₁-C₄-Alkylsulfonyl oder für jeweils durch Fluor oder Chlor substituiertes C₁-C₄-Alkyl oder C₁-C₄-Alkoxy.
- Z: steht besonders bevorzugt für Fluor, Chlor, Brom, Nitro, Cyano, C₁-C₆-Alkyl, C₁-C₄-Alkoxy, C₂-C₄-Alkenyloxy oder jeweils durch Fluor oder Chlor substituiertes C₁-C₄-Alkyl oder C₁-C₄-Alkoxy.
- n: steht besonders bevorzugt für eine der Zahlen 0, 1 oder 2.
- A: steht besonders bevorzugt für Wasserstoff, Fluor, Chlor oder Brom, für einen gegebenenfalls durch Fluor, Chlor oder Brom substituierten Rest aus der Reihe C₁-C₈-Alkyl, C₃-C₈-Cycloalkyl, C₃-C₈-Alkenyl und C₃-C₈-Alkinyl, für einen gegebenenfalls durch Fluor, Chlor, Brom, Nitro, C₁-C₄-Alkyl, C₁-C₄-Alkoxy, C₁-C₄-Alkylthio, C₁-C₄-Halogenalkyl, C₁-C₄-Halogenalkoxy oder Cyano substituierten Rest aus der Reihe Phenyl, Naphthyl, Furanyl, Thienyl, Pyridyl, Phenyl-C₁-C₄-alkyl, Furanyl-C₁-C₄-alkyl, Thienyl-C₁-C₄-alkyl und Pyridyl-C₁-C₄-alkyl oder für eine der Gruppen -COR³, -CO₂R³, -CN, -CONR³R⁴, -SO₂R³ oder -P(O)(OR³)OR⁴, worin
R³ und R⁴ unabhängig voneinander für Wasserstoff, für jeweils gegebenenfalls durch Fluor, Chlor oder Brom substituiertes C₁-C₈-Alkyl oder C₃-C₈-Alkenyl oder für einen gegebenenfalls durch Fluor, Chlor, Brom, Nitro, C₁-C₄-Alkyl, C₁-C₄-Alkoxy, C₁-C₄-Alkylthio, C₁-C₄-Halogenalkyl, C₁-C₄-Halogenalkoxy oder Cyano substituierten Rest aus der Reihe Phenyl, Furanyl, Thienyl, Pyridyl, Phenyl-C₁-C₄-alkyl, Furanyl-C₁-C₄-alkyl, Thienyl-C₁-C₄-alkyl und Pyridyl-C₁-C₄-alkyl stehen oder
R³ und R⁴ gemeinsam für eine C₂-C₆-Alkylen-Gruppe stehen, in welcher gegebenenfalls eine nicht endständige Methylengruppe durch Sauerstoff, Schwefel, NH oder N-C₁-C₄-Alkyl ersetzt ist.
- D: steht besonders bevorzugt für Wasserstoff, für jeweils gegebenenfalls durch Fluor oder Chlor substituiertes C₁-C₁₀-Alkyl, C₃-C₆-Alkenyl, C₃-C₆-Alkinyl, C₁-C₈-Alkoxy-C₂-C₆-alkyl, Poly-C₁-C₆-alkoxy-C₂-C₆-alkyl oder C₁-C₈-Alkylthio-C₂-C₆-alkyl, für durch Cyano, C₁-C₆-Alkoxycarbonyl oder C₁-C₆-Alkylcarbonyloxy substituiertes C₁-C₈-Alkyl, für gegebenenfalls durch Fluor, Chlor, C₁-C₄-Alkyl, C₁-C₄-Alkoxy oder C₁-C₂-Halogenalkyl substituiertes C₃-C₇-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, C₁-C₄-Alkyl, C₁-C₄-Halogenalkyl, C₁-C₄-Alkoxy, C₁-C₄-Halogenalkoxy, Cyano oder Nitro substituiertes Phenyl, Furanyl, Imidazolyl, Pyridyl, Thiazolyl, Pyrazolyl, Pyrimidyl, Pyridazyl, Pyrazinyl, Pyrrolyl, Thienyl, Triazolyl oder Phenyl-C₁-C₄-alkyl oder
- A und D: stehen gemeinsam besonders bevorzugt für eine jeweils gegebenenfalls durch C₁-C₄-Alkyl, Fluor, Chlor oder Brom substituierte C₂-C₆-Alkylen- oder C₂-C₆-Alkenylen-Gruppe, worin gegebenenfalls zwei Kohlenstoffatome durch eine C₁-C₂-Alkylengruppe verbunden sind und in welcher gegebenenfalls eine Methylengruppe durch Sauerstoff, Schwefel, NH, N-C₁-C₄-Alkyl oder die Gruppe -O-CO- ersetzt ist.
- R¹: steht besonders bevorzugt für Wasserstoff oder für gegebenenfalls durch Fluor, Chlor oder Brom substituiertes C₁-C₄-Alkyl.
- R²: steht besonders bevorzugt für jeweils gegebenenfalls durch Fluor, Chlor oder Brom substituiertes C₁-C₈-Alkyl, C₃-C₈-Alkenyl oder C₃-C₈-Alkinyl.
- X: steht ganz besonders bevorzugt für Fluor, Chlor, Brom, Nitro, Cyano, Methyl, Ethyl, n- oder i-Propyl, n-, s-, i- oder t-Butyl, Vinyl, Allyl, Methallyl, Methoxy, Ethoxy, n- oder i-Propoxy, Allyloxy, Methallyloxy, Trifluormethyl, Difluormethoxy, Trifluormethoxy, Trifluorethoxy, Methylthio, Methylsulfinyl oder Methylsulfonyl.
- Y: steht ganz besonders bevorzugt für Wasserstoff, Fluor, Chlor, Brom, Nitro, Cyano, Methyl, Ethyl, n- oder i-Propyl, n-, s-, i- oder t-Butyl, Methoxy, Ethoxy, n- oder i-Propoxy, Allyloxy, Methallyloxy, Trifluormethyl, Difluormethoxy, Trifluormethoxy, Trifluorethoxy, Methylthio, Methylsulfinyl oder Methylsulfonyl.
- Z: steht ganz besonders bevorzugt für Fluor, Chlor, Brom, Nitro, Cyano, Methyl, Ethyl, n- oder i-Propyl, n-, s-, i- oder t-Butyl, Methoxy, Ethoxy, n-oder i-Propoxy, Allyloxy, Methallyloxy, Trifluormethyl, Difluormethoxy, Trifluormethoxy oder Trifluorethoxy.
- n: steht ganz besonders bevorzugt für eine der Zahlen 0 oder 1.
- A: steht ganz besonders bevorzugt für Wasserstoff, Fluor, Chlor oder Brom, für einen gegebenenfalls durch Fluor oder Chlor substituierten Rest aus der Reihe C₁-C₆-Alkyl, C₃-C₆-Cycloalkyl, C₃-C₆-Alkenyl und C₃-C₆-Alkinyl, für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl, Furanyl, Pyridyl, Thienyl oder Benzyl oder für eine der Gruppen -COR³, -CO₂R³, -CN, -CONR³R⁴, -SO₂R³ oder -P(O)(OR³)OR⁴, worin
R³ und R⁴ unabhängig voneinander für Wasserstoff, für jeweils gegebenenfalls durch Fluor oder Chlor substituiertes C₁-C₆-Alkyl oder C₃-C₆-Alkenyl oder für gegebenenfalls durch Fluor, Chlor, Brom, Nitro, C₁-C₄-Alkyl, C₁-C₄-Alkoxy, C₁-C₄-Alkylthio, C₁-C₄-Halogenalkyl, C₁-C₄-Halogenalkoxy oder Cyano substituiertes Phenyl stehen oder
R³ und R⁴ gemeinsam für eine der Gruppen -(CH₂)₃-, -(CH₂)₄-, -(CH₂)₅-, -CH₂-CH(CH₃)(CH₂)₂-, -CH₂O-(CH₂)₂- und -CH₂S-(CH₂)₂- stehen.
- D: steht ganz besonders bevorzugt für Wasserstoff, für jeweils gegebenenfalls durch Fluor oder Chlor substituiertes C₁-C₈-Alkyl, C₃-C₄-Alkenyl, C₃-C₄-Alkinyl, C₁-C₆-Alkoxy-C₂-C₄-alkyl, Poly-C₁-C₄-alkoxy-C₂-C₄-alkyl oder C₁-C₄-Alkylthio-C₂-C₄-alkyl oder für gegebenenfalls durch Fluor, Chlor, Methyl oder Ethyl substituiertes C₃-C₆-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl, Furanyl, Pyridyl, Thienyl oder Benzyl oder
- A und D: stehen gemeinsam ganz besonders bevorzugt für eine der Gruppen -(CH₂)₃-, -(CH₂)₄-, -(CH₂)₅-, -CH₂-CH(CH₃)(CH₂)₂-, -CH₂O-(CH₂)₂-, -CH₂S-(CH₂)₂- oder 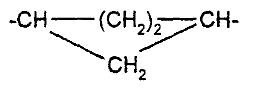
- R¹: steht ganz besonders bevorzugt für Wasserstoff oder für gegebenenfalls durch Fluor oder Chlor substituiertes Methyl oder Ethyl.
- R²: steht ganz besonders bevorzugt für jeweils gegebenenfalls durch Fluor oder Chlor substituiertes Methyl, Ethyl, n- oder i-Propyl, n-, i-, s- oder t-Butyl, Allyl, Methallyl, Butenyl, Propargyl oder Butinyl.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Verwendet man beispielsweise 5-Fluor-3-[4-(2-brom-4,6-dimethyl-phenyl)-4-hydroxy-6-methyl]-2-pyron und Propargyloxychlormethan als Ausgangsverbindungen, so kann der Verlauf des erfindungsgemäßen Verfahrens durch das folgende Reaktionsschema wiedergegeben werden:

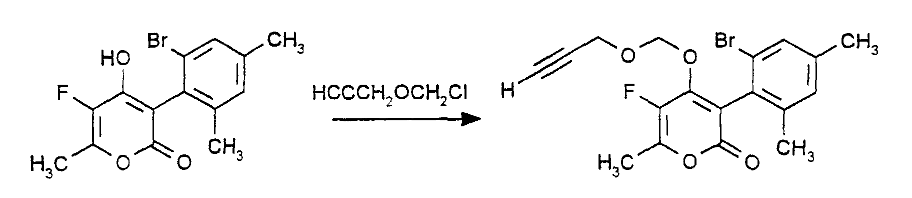

Die beim erfindungsgemäßen Verfahren als Ausgangsstoffe benötigten 4-Hydroxy-2-pyrone der Formel (II) sind teilweise neu. Sie sind in der EP-A-588 137 beschrieben oder Gegenstand einer vorgängigen, noch nicht veröffentlichten Anmeldung der Anmelderin (Deutsche Patentanmeldung mit dem Aktenzeichen 195 40 080.1 vom 27.10.1995).

Die beim erfindungsgemäßen Verfahren als Ausgangsstoffe benötigten Verbindungen der Formel (III) sind käufliche, allgemein bekannte oder nach bekannten Verfahren zugängliche Verbindungen.

Das erfindungsgemäßen Verfahren ist dadurch gekennzeichnet, daß man Verbindungen der Formel (II) mit α-Alkoxyhalogeniden der Formel (III) gegebenenfalls in Gegenwart eines Verdünnungsmittels und gegebenenfalls in Gegenwart eines Reaktionshilfsmittels umsetzt.

Als Verdünnungsmittel können bei dem erfindungsgemäßen Verfahren alle gegenüber den Halogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, Nitrile wie Acetonitril und auch stark polare Solventien, wie Dimethylformamid, Dimethylsulfoxid und Sulfolan. Wenn die Hydrolysestabilität des Halogenids es zuläßt, kann die Umsetzung auch in Gegenwart von Wasser durchgeführt werden.

Als Reaktionshilfsmittel bei der Umsetzung nach dem erfindungsgemäßen Verfahren eignen sich beispielsweise alle üblichen Säureakzeptoren. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Die Reaktionstemperatur kann bei dem erfindungsgemäßen Verfahren innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens werden der Ausgangsstoff der Formel (II) und das Halogenid der Formel (III) im allgemeinen jeweils in angenähert äquivalenten Mengen verwendet. Es ist jedoch auch möglich, das Halogenid in einem größeren Überschuß (bis zu 5 Mol) einzusetzen.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der Organischen Chemie. Die Endprodukte werden vorzugsweise durch Kristallisation, chromatographische Reinigung oder durch sogenanntes "Andestillieren", d.h. Entfernung der flüchtigen Bestandteile im Vakuum gereinigt.

Die erfindungsgemäßen Verbindungen eignen sich bei guter Pflanzenverträglichkeit und günstiger Warmblütertoxizität zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp..
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium comi, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima. Tortrix viridana.

Aus der Ordnung der Coleoptera z.B. Anobium punctatum, Rhizopertha dominica, Acanthoscelides obtectus, Bruchidius obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit aus.

Sie lassen sich mit besonders gutem Erfolg einsetzen zur Bekämpfung von pflanzenschädigenden Insekten, wie beispielsweise gegen die Larven des Meerrettichblattkäfers (Phaedon cochleariae), gegen die Raupen der Kohlschabe (Plutella maculipennis), gegen die Raupen des Eulenfalters (Spodoptera frugiperda), gegen die Larven der grünen Reiszikade (Nephotettix cincticeps), gegen Pfirsichblattläuse (Myzus persicae) oder gegen schwarze Bohnenblattläuse (Aphis fabae) und zur Bekämpfung von pflanzenschädigenden Spinnentieren (Acari) wie beispielsweise gegen die gemeine Spinnmilbe (Tetranychus urticae).

Die erfindungsgemäßen Wirkstoffe weisen eine stark mikrobizide Wirkung auf und können zur Bekämpfung von unerwünschten Mikroorganismen praktisch eingesetzt werden. Die Wirkstoffe sind auch für den Gebrauch als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei werden die erfindungsgemäßen Wirkstoffe mit besonders gutem Erfolg zur Behandlung von Befall durch den Pilz Sphaerotheca fuliginea, den Apfelschorferregers (Venturia inaequalis) und der Pilze Botrytis cinerea sowie Pyricularia oryzae sowie ferner Uncinula necator und Podosphaera leucotricha verwendet.

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum. Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenociea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich zur Bekämpfung von monokotylen und dikotylen Unkräutern in monokotylen und dikotylen Kulturen sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, kömige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

### Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Dichlofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfemaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

**Bakterizide:**
Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

**Insektizide / Akarizide / Nematizide:**
Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin. BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Cloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonofos, Formothion, Fosthiazat, Fubfenprox, Furathiocarb,
HCH, Heptenophos, Hexaflumuron, Hexythiazox,
Imidacloprid, Iprobenfos, Isazofos, Isofenphos, Isoprocarb, Isoxathion, Ivermectin, Lamda-cyhalothrin, Lufenuron,
Malathion, Mecarbam, Mevinphos, Mesulfenfos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram,
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Primiphos A, Profenofos, Promecarb, Propaphos, Propoxur, Prothiofos, Prothoat, Pymetrozin, Pyraclofos, Pyridaphenthion, Pyresmethrin, Pyrethrum, Pyridaben, Pyrimidifen, Pyriproxyfen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

**Herbizide:**
beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon: Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfüron, Metsulfuron-methyl, Nicosulfuron, Primisulfuron, Pyrazosulfuronethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit Düngemitteln und Wachstumsregulatoren ist möglich.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise. Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Omithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine hervorragende Wirksamkeit gegen Lucilia cuprina.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen

Formkörpem, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:

### Käfer wie

Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.

### Hautflügler wie

Sirex juvencus, Urocerus gigas, Urocerus gigas taignus, Urocerus augur.

### Termiten wie

Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

### Borstenschwänze

wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/ oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlomaphthalin, vorzugsweise a-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/ oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyrifos, Phoxim, Silafluofen, Alphamethrin, Cyfluthrin, Cypermethrin, Deltamethrin, Permethrin, Imidacloprid, NI-25, Flufenoxuron, Hexaflumuron und Triflumuron, sowie Fungizide wie Epoxyconazole, Hexaconazole, Azaconazole, Propiconazole, Tebuconazole, Cyproconazole, Metconazole, Imazalil, Dichlofluanid, Tolylfiuanid, 3-Iod-2-propinyl-butylcarbamat, N-Octyl-isothiazolin-3-on und 4,5-Dichlor-N-octylisothiazolin-3-on genannt.

Die Herstellung und die Verwendung der erfindungsgemäßen Wirkstoffe geht aus den nachfolgenden Beispielen hervor.

### Herstellungsbeispiele

### Beispiel 1

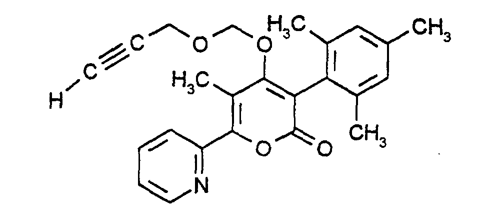

Unter Feuchtigkeitsausschluß legt man 6,4 g (20 mmol) 4-Hydroxy-5-methyl-6-(2-pyridyl)-3-(2,4,6-trimethylphenyl)-2-pyron in 50 ml Ethylacetat vor. Dazu gibt man bei 20 °C 2,02 g (20 mmol) Triethylamin und tropft bei 0 °C unter Kühlung eine Lösung von 2,1 g (20 mmol) Propargyloxychlormethan in 20 ml Ethylacetat zu. Anschließend wird 20 h bei 20 °C nachgerührt und der Umsatz dünnschichtchromatographisch verfolgt. Man saugt vom ausgefallenen Triethylamin-hydrochlorid ab und wäscht mit Ethylacetat. Die vereinigten Mutterlaugen werden zweimal mit je 50 ml halbgesättigter Kochsalzlösung gewaschen, über Magnesiumsulfat getrocknet und im Vakuum eingedampft. Man erhält 8 g Rohprodukt. Durch "Flash"-Chromatographie an 500 g Kieselgel 60 (35-70 µm) mit Toluol:Aceton 20:1 als Laufmittel erhält man 6,4 g (82 % der Theorie) 5-Methyl-6-(2-pyridyl)-4-propargyloxymethoxy-3-(2,4,6-trimethylphenyl)-2-pyron als Öl.
¹H-NMR (CDCl₃) δ [ppm]: 8,70 (m, 1H); 7,94 (td, 1H); 7,83 (m, 1H); 7,34 (m, 1H); 6,93 (s, 2H); 4,74 (s, 2H); 4,11 (d, 2H); 2,42 (t, 1H); 2,41 (s, 3H); 2,30 (s, 3H); 2,20 (s, 6H).

### Beispiel 2

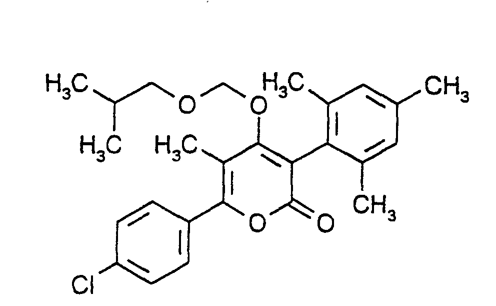

Unter Feuchtigkeitsausschluß legt man 7,1 g (20 mmol) 6-(4-Chlorphenyl)-4-hydroxy-5-methyl-3-(2,4,6-trimethylphenyl)-2-pyron in 50 ml Ethylacetat vor. Dazu gibt man bei 20 °C 2,02 g (20 mmol) Triethylamin und tropft unter Kühlung bei 0 °C eine Lösung von 2,4 g (20 mmol) i-Butoxychlormethan in 20 ml Ethylacetat zu. Anschließend wird 20 h bei 20 °C nachgerührt und der Umsatz dünnschichtchromatographisch verfolgt. Man saugt vom ausgefallenen Triethylamin-hydrochlorid ab und wäscht mit Ethylacetat. Die vereinigten Mutterlaugen werden zweimal mit je 50 ml halbgesättigter Kochsalzlösung gewaschen, über Magnesiumsulfat getrocknet und im Vakuum eingedampft. Man erhält 8,8 g Rohprodukt. Durch "Flash"-Chromatographie an 500 g Kieselgel 60 (35-70 µm) mit Toluol: Aceton 50:1 als Laufmittel erhält man 5,6 g (63 % der Theorie) 6-(4-Chlorphenyl)-4-[(2-methyl-1-propoxy)-methoxy]-5-methyl-3-(2,4,6-trimethylphenyl)-2-pyron als Öl.
¹H-NMR (CDCl₃) δ [ppm]: 7,52 (AA'BB', 4H); 6,92 (s, 2H); 4,57 (s, 2H); 3,26 (d, 2H); 2,30 (s, 3H); 2,20 (s, 6H); 2,15 (s, 3H); 1,78 (m, 1H); 0,87 (d, 6H).

### Beipiele 3 - 35

Analog zu Beispiel 1 und 2 bzw. gemäß den allgemeinen Angaben zur Herstellung wurden die in der nachfolgenden Tabelle 1 aufgeführten Verbindungen der Formel (I-a) erhalten.

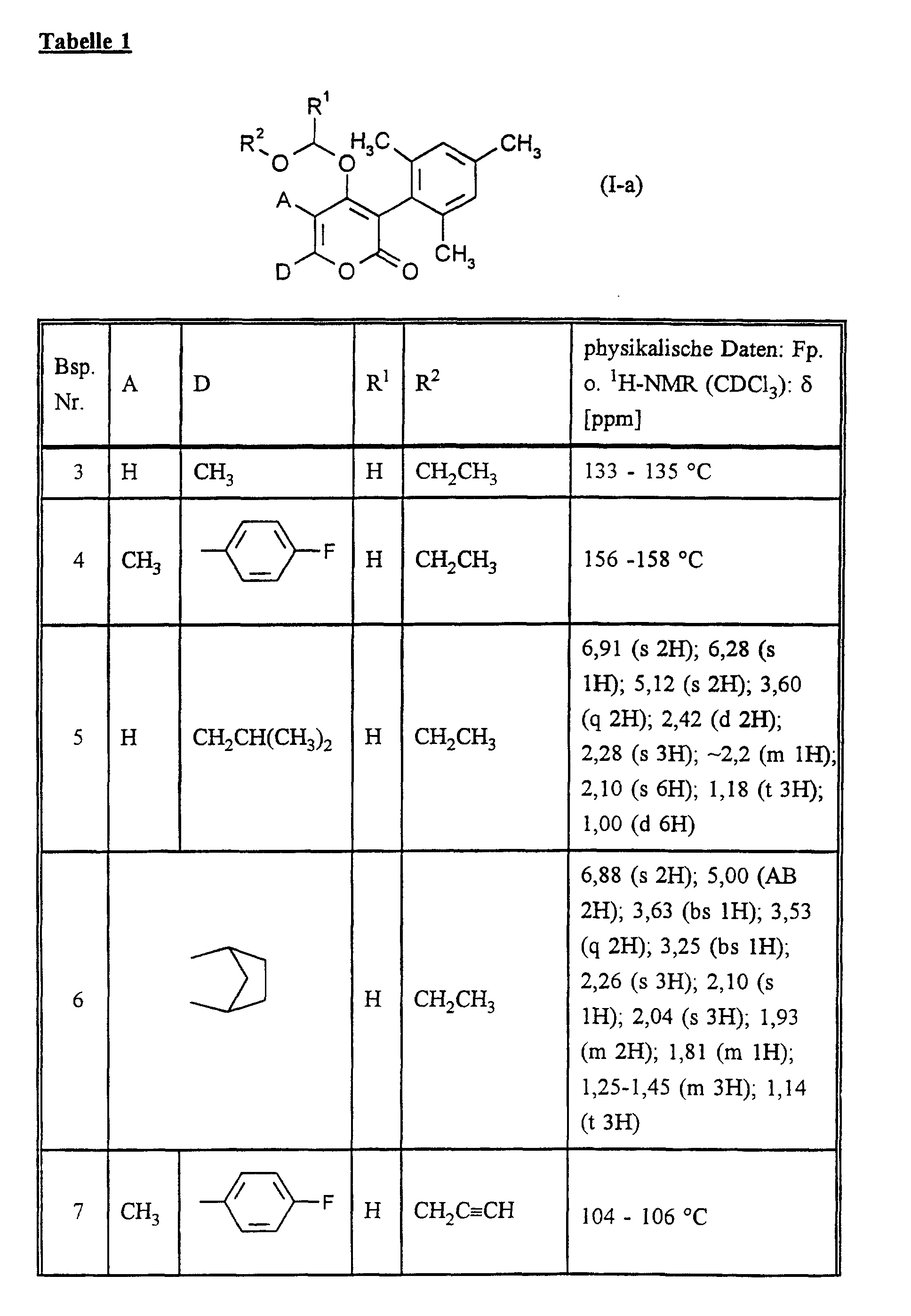

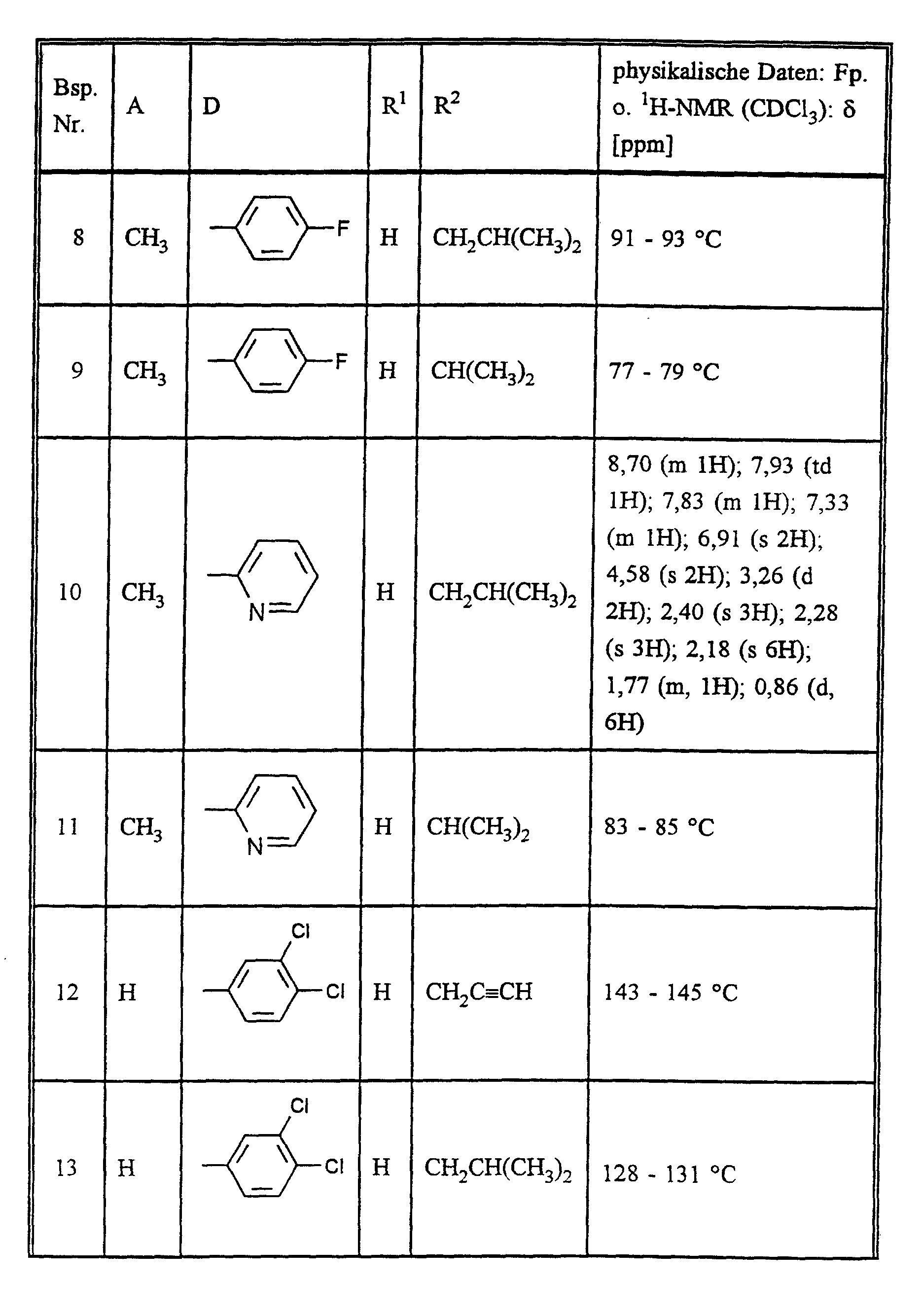

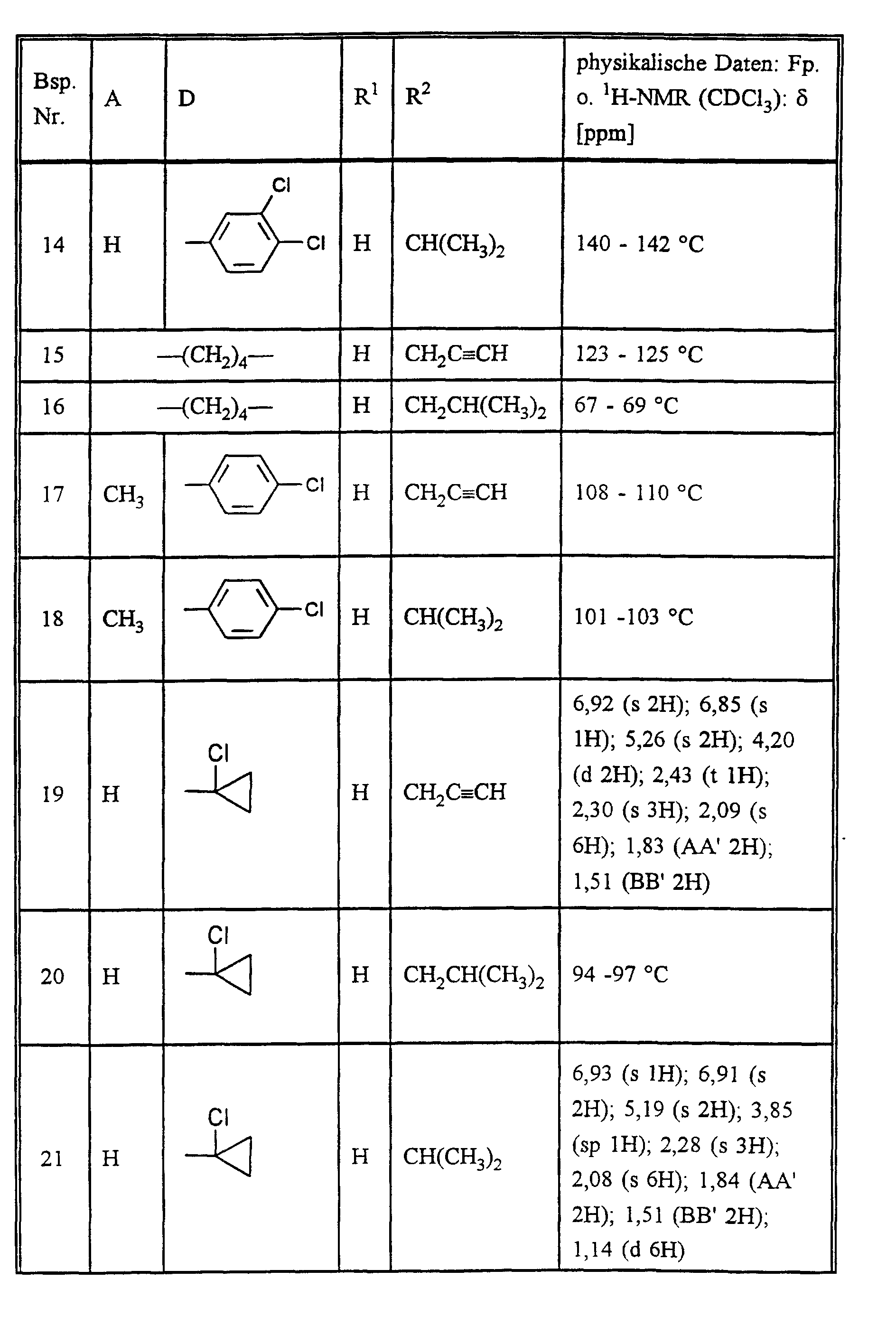

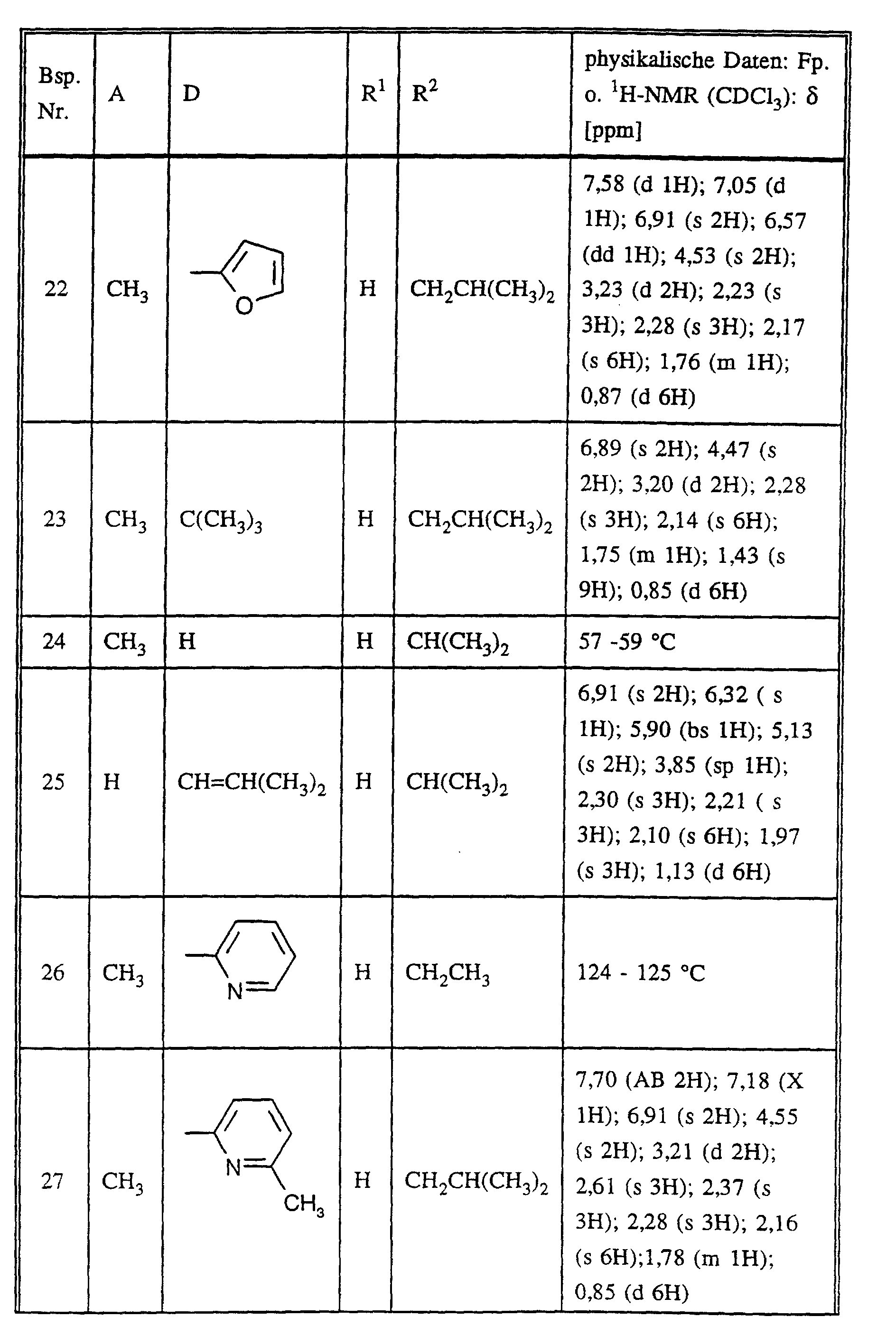

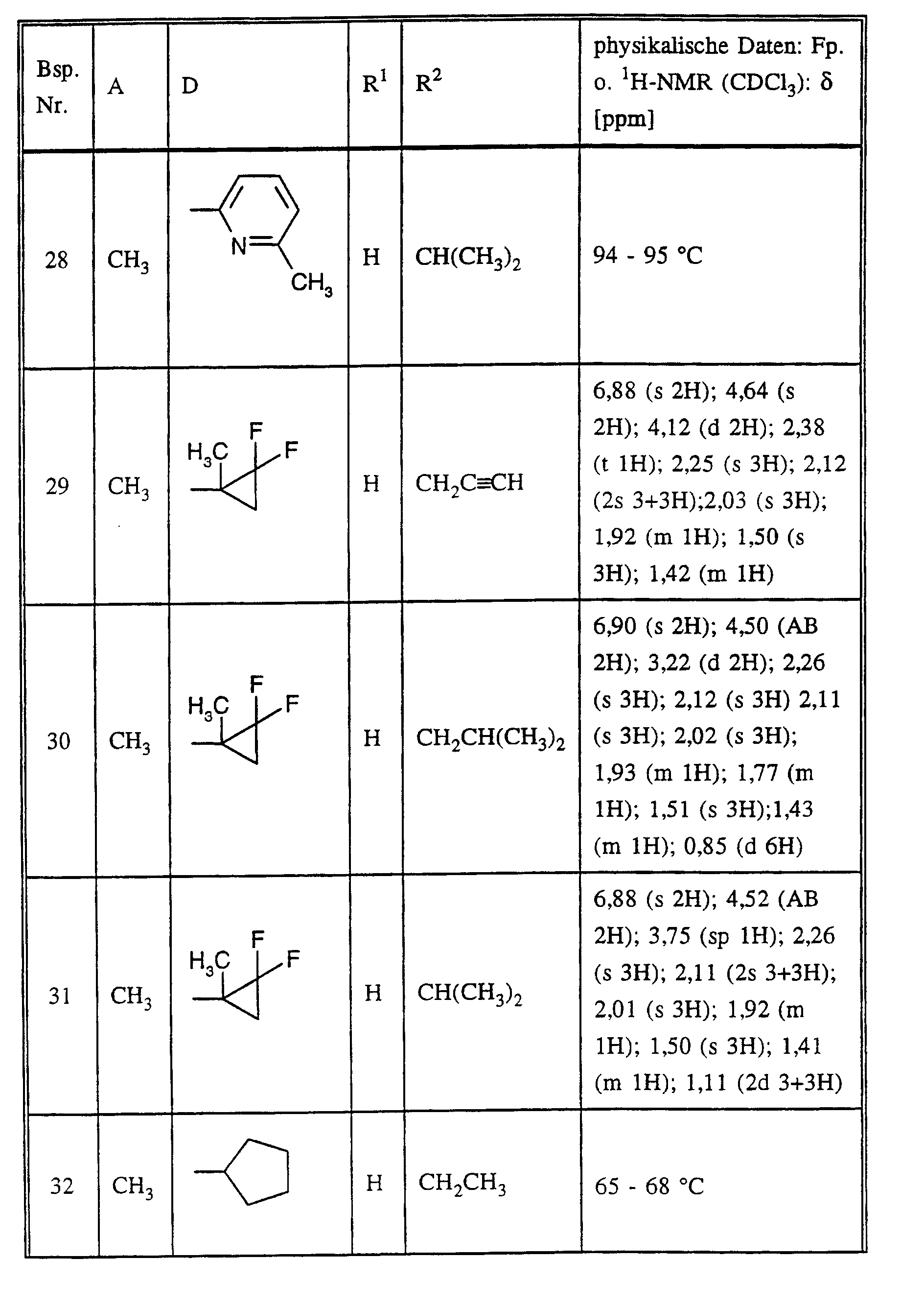

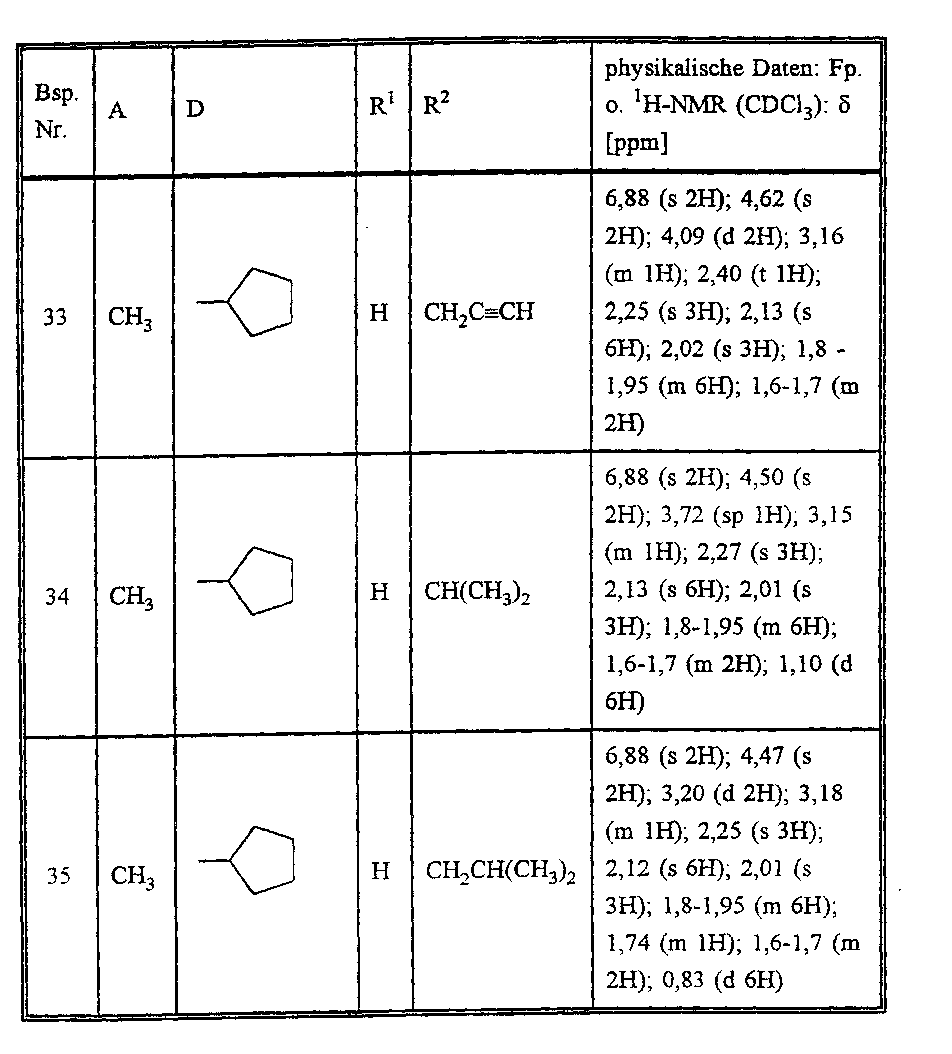

### Beispiel 36

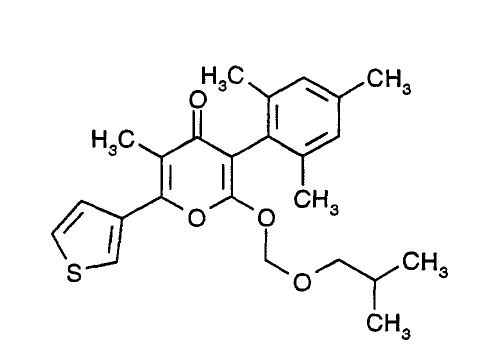

Analog Beispiel 1 erhält man aus 4-Hydroxy-5-methyl-6-(3-thienyl)-3-(2,4,6-trimethylphenyl)-2-pyron und i-Butoxychlormethan bei 102-104 °C schmelzendes 2-[(2-Methyl-1-propoxy)-methoxy]-5-methyl-6-(3-thienyl)-3-(2,4,6-trimethylphenyl)-4-pyron

### Beispiele 37 - 43

Analog zu Beispiel 1 und 2 bzw. gemäß den allgemeinen Angaben zur Herstellung wurden die in der nachfolgenden Tabelle 2 aufgeführten Verbindungen der Formel (I-b) erhalten.

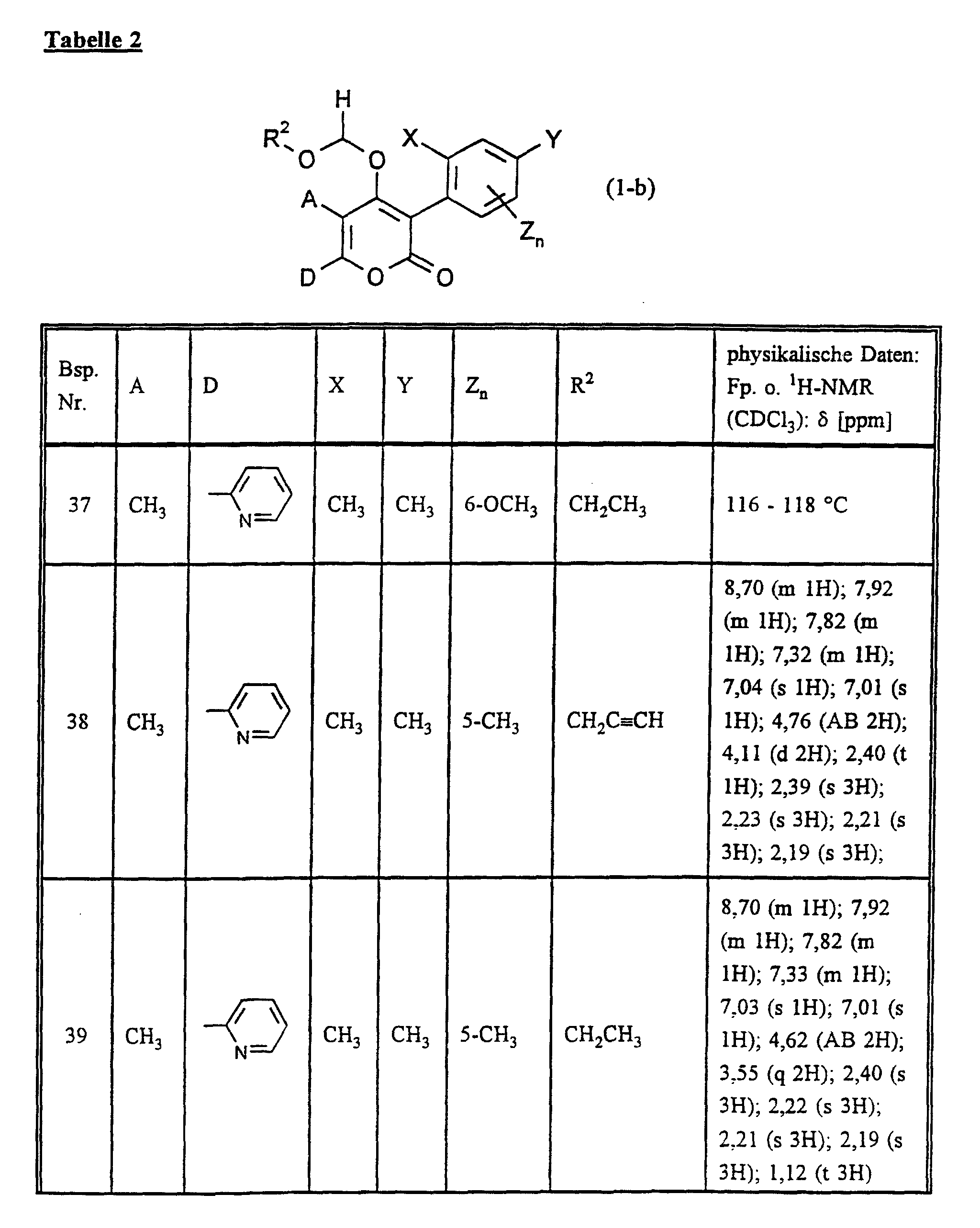

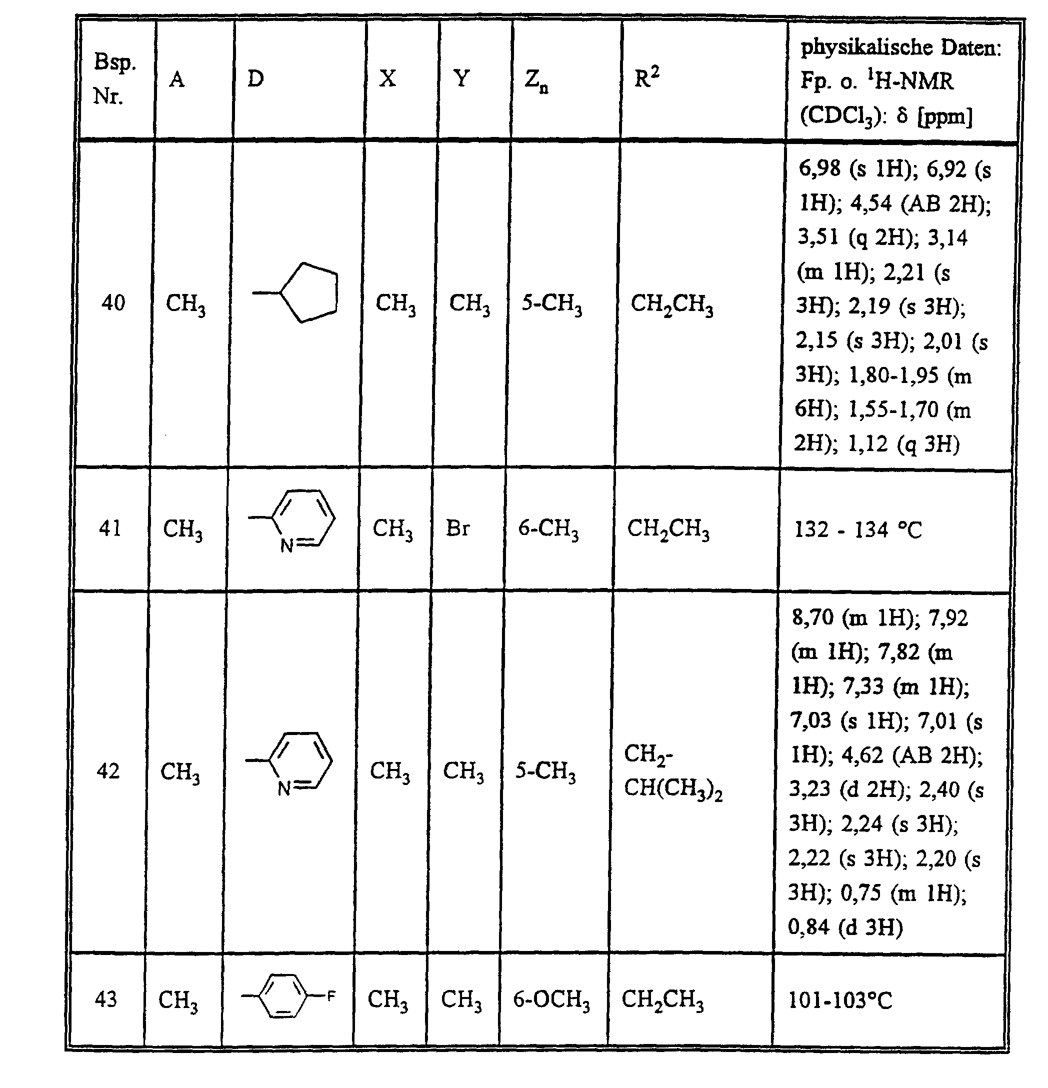

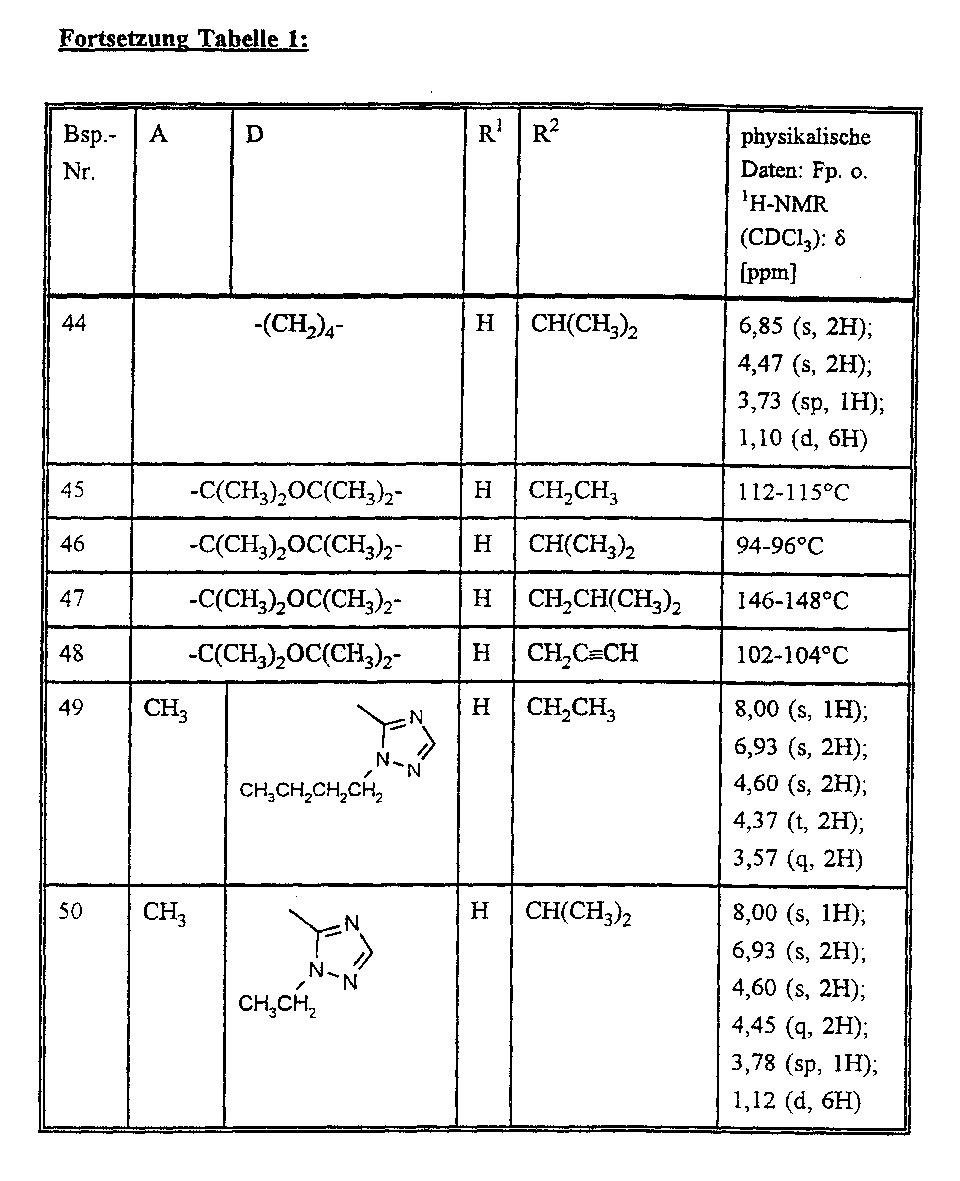

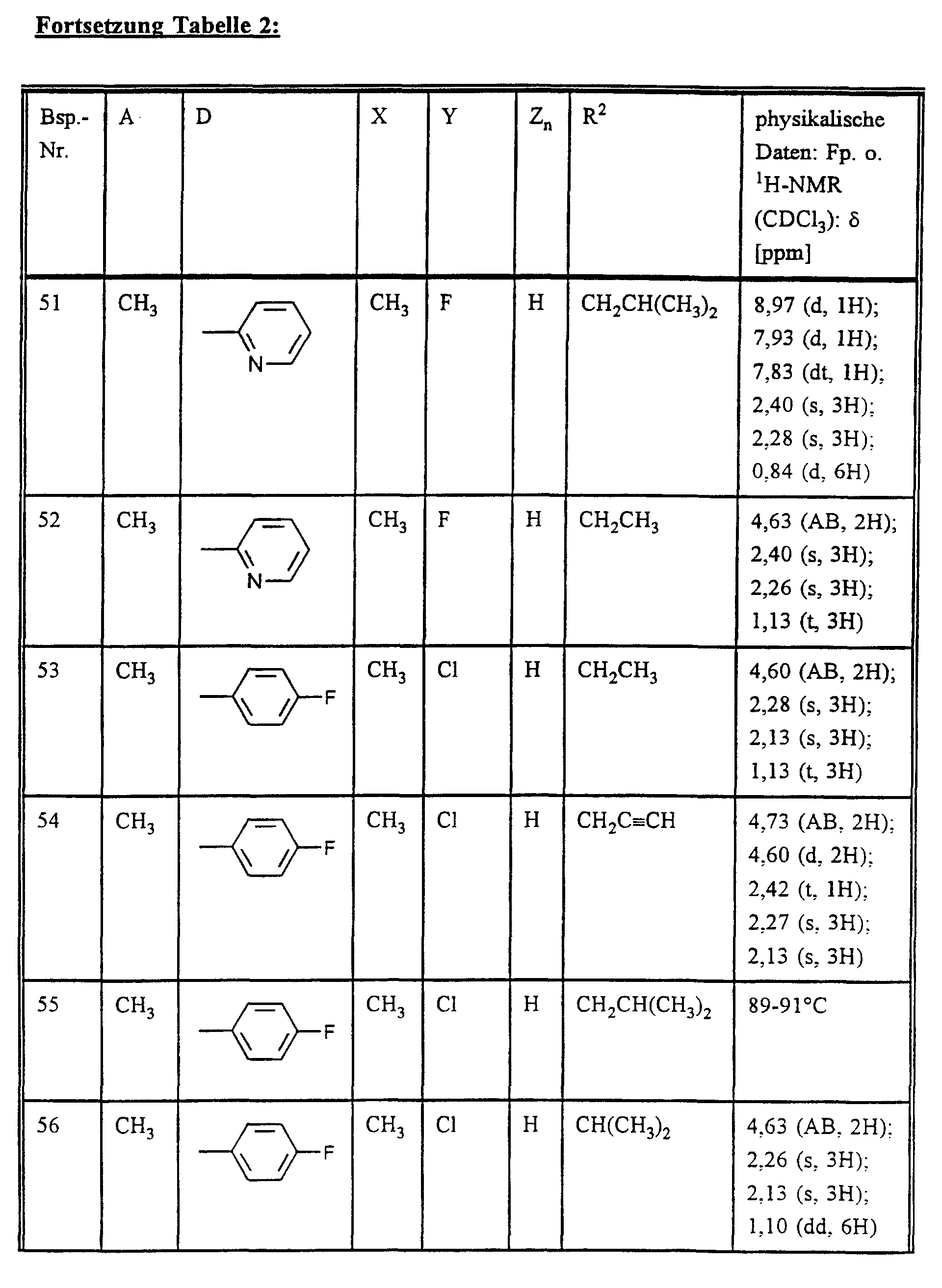

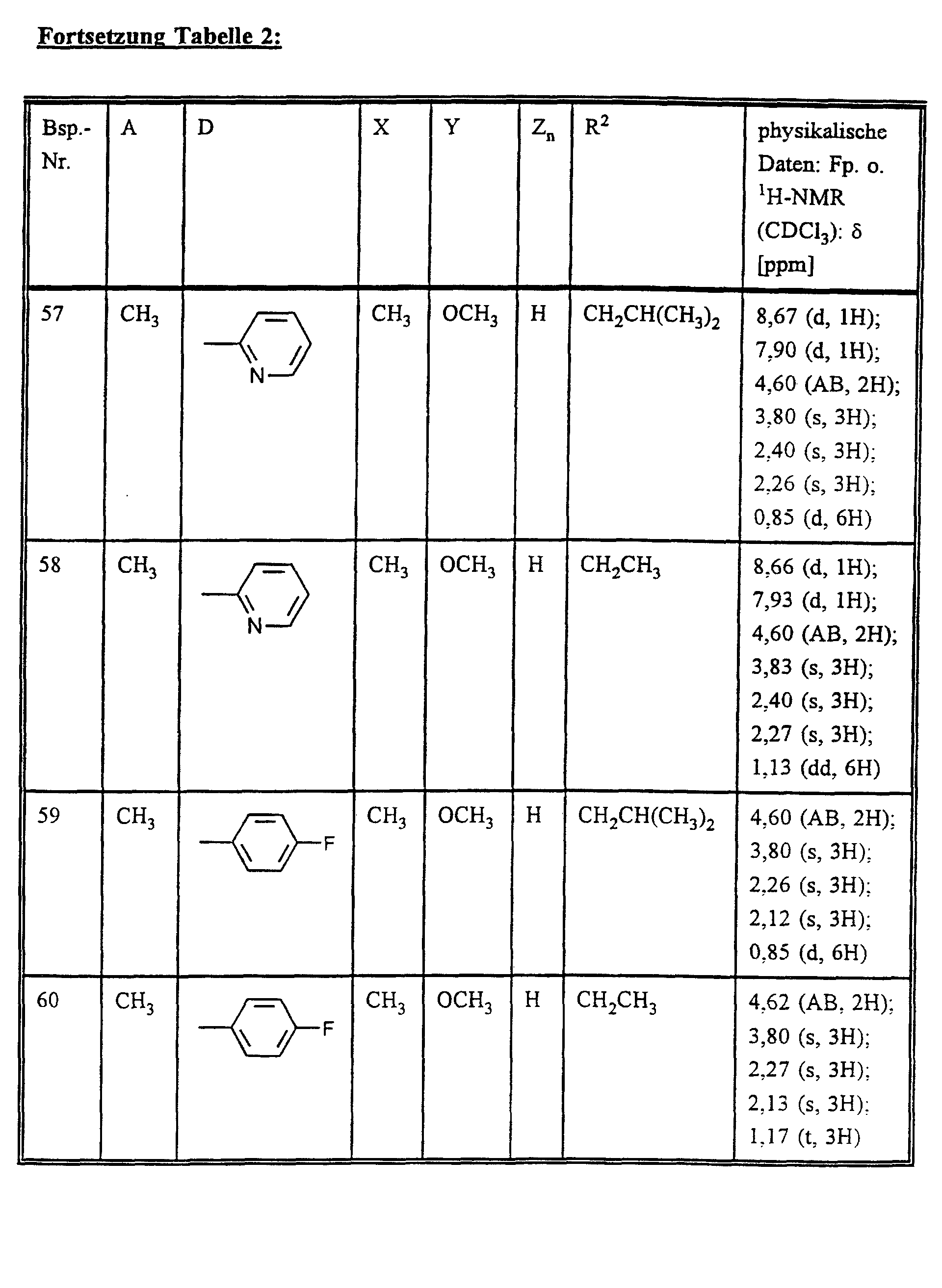

### Anwendungsbeispiele

### Beispiel A

### Phaedon-Larven-Test

| | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test zeigten z.B. die Verbindungen gemäß der Herstellungsbeispiele 4, 5, 9, 26, 27, 28 und 31 bei einer beispielhaften Wirkstoffkonzentration von 0,01 % nach 7 Tagen einen Abtötungsgrad von 100 %.

### Beispiel B

### Plutella-Test

| | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigten z.B. die Verbindungen gemäß der Herstellungsbeispiele 4, 5, 9, 10, 18, 27, 28, 31, 32 und 34 bei einer beispielhaften Wirkstoffkonzentration von 0,1 % nach 7 Tagen einen Abtötungsgrad von 100 %.

### Beispiel C

### Spodoptera-Test

| | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigten z.B. die Verbindungen gemäß der Herstellungsbeispiele 4, 5, 9, 32 und 34 bei einer beispielhaften Wirkstoffkonzentration von 0,1 % nach 7 Tagen einen Abtötungsgrad von 100 %.

### Beispiel D

### Nephotettix-Test

| | | |
|---|---|---|
| Lösungsmittel | 7 Gewichtsteile | Dimethylforamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryzae sativa) werden durch Tauchen in die Wirkstoffzubereitung der gewünschten Konzentration behandelt und mit Larven der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test zeigten z.B. die Verbindungen gemäß den Herstellungsbeispielen 1, 4, 7, 9, 11, 18, 21, 26, 27, 28, 29, 30, 31, 32 und 33 bei einer beispielhaften Wirkstoffkonzentration von 0,1 % nach 6 Tagen einen Abtötungsgrad von 100 %.

### Beispiel E

### Myzus-Test

| | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigten z.B. die Verbindungen gemäß der Herstellungsbeispiele 1, 9, 11, 26, 27, 31, 32 und 36 bei einer beispielhaften Wirkstoffkonzentration von 0,1 % nach 6 Tagen einen Abtötungsgrad von 95 bis 100 %.

### Beispiel F

### Grenzkonzentrations-Test / Wurzelsystemische Wirkung

| | |
|---|---|
| Testinsekt | Aphis fabae |
| Lösungsmittel | 4 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Bohnen (Vicia faba). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit schwarzen Bohnenblattläusen (Aphis fabae) besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigten z.B. die Verbindungen gemäß der Herstellungsbeispiele 1, 10 und 11 bei einer beispielhaften Wirkstoffkonzentration von 20 ppm einen Abtötungsgrad von 100 %.

### Beispiel G

### Tetranychus-Test (resistent)

| | | |
|---|---|---|
| Lösungsmittel | 3 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigten z.B. die Verbindungen gemäß der Herstellungsbeispiele 9, 10, 11, 21 und 30 bei einer beispielhaften Wirkstoffkonzentration von 0,01 % nach 13 Tagen einen Abtötungsgrad von 100 %.

### Beispiel H

### Sphaerotheca-Test (Gurke) / protektiv

| | | |
|---|---|---|
| Lösungsmittel | 4,7 Gewichtsteile | Aceton |
| Emulgator | 0,3 Gewichtsteile | Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung.

Bei diesem Test zeigten z.B. die Verbindungen gemäß der Herstellungsbeispiele 1, 2, 4, 7, 8, 9, 10, 11, 17, 18 und 21 bei einer beispielhaften Wirkstoffkonzentration von 100 ppm im Vergleich zur unbehandelten Kontrolle einen Wirkungsgrad von 100 %.

### Beispiel I

### Venturia-Test (Apfel) / protektiv

| | | |
|---|---|---|
| Lösungsmittel | 4,7 Gewichtsteile | Aceton |
| Emulgator | 0,3 Gewichtsteile | Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Bei diesem Test zeigten z.B. die Verbindungen gemäß der Herstellungsbeispiele 4, 9 und 18 bei einer beispielhaften Wirkstoffkonzentration von 100 ppm im Vergleich zur unbehandelten Kontrolle einen Wirkungsgrad von 95 - 99 %.

### Beispiel J

### Botrytis-Test (Bohne) / protektiv

| | | |
|---|---|---|
| Lösungsmittel | 4,7 Gewichtsteile | Aceton |
| Emulgator | 0,3 Gewichtsteile | Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Bei diesem Test zeigten z.B. die Verbindungen gemäß der Herstellungsbeispiele 4, 9 und 18 bei einer beispielhaften Wirkstoffkonzentration von 500 ppm im Vergleich zur unbehandelten Kontrolle einen Wirkungsgrad von 94 - 99 %.

### Beispiel K

### Pyricularia-Test (Reis) / protektiv

| | | |
|---|---|---|
| Lösungsmittel | 12,5 | Gewichtsteile Aceton |
| Emulgator | 0,3 | Gewichtsteile Alkyl-Aryl-Polyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. 4 Tage nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Bei diesem Test zeigten z.B. die Verbindungen gemäß der Herstellungsbeispiele 4, 5, 8, 9, 10, 11 und 18 bei einer beispielhaften Wirkstoffkonzentration von 0,05 % im Vergleich zur unbehandelten Kontrolle einen Wirkungsgrad von 70 - 100 %.

### Beispiel L

### Test mit Fliegenlarven / Entwicklungshemmende Wirkung

| | |
|---|---|
| Testtiere | Alle larvalen Stadien von Lucilia cuprina (OP-resistent) [Puppen und Adulte (ohne Kontakt zum Wirkstoff)] |
| Lösungsmittel | 35 Gewichtsteile Ethylenglykolmonomethylether 35 Gewichtsteile Nonylphenolpolyglykolether |

Zwecks Herstellung einer geeigneten Formulierung vermischt man 3 Gewichtsteile Wirkstoff mit 7 Teilen des oben angegebenen Lösungsmittel-Emulgator-Gemisches und verdünnt das so erhaltene Emulsionskonzentrat mit Wasser auf die jeweils gewünschte Konzentration.

30 bis 50 Larven je Konzentration werden auf in Glasröhrchen befindliches Pferdefleisch (1 cm³) gebracht, auf welches 500 µl der zu testenden Verdünnung pipettiert werden. Die Glasröhrchen werden in Kunststoffbecher gestellt, deren Boden mit Seesand bedeckt ist, und im klimatisierten Raum (26°C ± 1,5°C, 70 % rel. Feuchte ± 10 %) aufbewahrt. Die Wirkungskontrolle erfolgt nach 24 Stunden und 48 Stunden (larvizide Wirkung). Nach dem Auswandern der Larven (ca. 72 h) werden die Glasröhrchen entfernt und gelochte Kunststoffdeckel auf die Becher gesetzt. Nach 1½-facher Entwicklungsdauer (Schlupf der Kontrollfliegen) werden die geschlüpften Fliegen und die Puppen/Puppenhüllen ausgezählt.

Als Kriterium für die Wirkung gilt der Eintritt des Todes bei den behandelten Larven nach 48 h (larvizider Effekt), bzw. die Hemmung des Adultschlupfes aus den Puppen bzw. die Hemmung der Puppenbildung. Als Kriterium für die in-vitro-Wirkung einer Substanz gilt die Hemmung der Fliegenentwicklung, bzw. ein Entwicklungsstillstand vor dem Adulten-Stadium. Dabei bedeutet 100 % larvizide Wirkung, daß nach 48 Stunden alle Larven abgestoben sind. 100 % entwicklungsinhibitorische Wirkung bedeutet, daß keine adulte Fliegen geschlüpft sind.

In diesem Test hatten z.B. die Verbindungen gemäß den Herstellungsbeispielen 1, 4, 9, 10, 11 und 18 bei einer beispielhaften Wirkstoffkonzentration von 1000 ppm eine Wirkung von 100 %.

## Patentansprüche

1. Verbindungen der Formel (I) 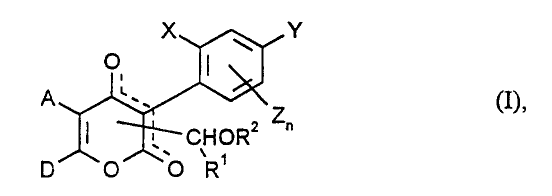 in welcher
X für Halogen, Nitro, Cyano, Alkyl, Alkenyl, Alkoxy, Alkenyloxy, Alkylthio, Alkylsulfinyl, Alkylsulfonyl, Halogenalkyl, Halogenalkenyl, Halogenalkoxy, Halogenalkenyloxy oder jeweils gegebenenfalls substituiertes Phenyl, Phenoxy, Phenylthio, Benzyloxy oder Benzylthio steht,
Y für Wasserstoff, Halogen, Nitro, Cyano, Alkyl, Alkenyl, Alkoxy, Alkenyloxy, Alkylthio, Alkylsulfinyl, Alkylsulfonyl, Halogenalkyl, Halogenalkenyl, Halogenalkoxy oder Halogenalkenyloxy steht,
Z für Halogen, Nitro, Cyano, Alkyl, Alkenyl, Alkoxy, Alkenyloxy, Halogenalkyl, Halogenalkenyl, Halogenalkoxy oder Halogenalkenyloxy steht,
n für eine der Zahlen 0, 1, 2 oder 3 steht,
A für Wasserstoff, Halogen, für einen gegebenenfalls substituierten Rest aus der Reihe Alkyl, Cycloalkyl, Alkenyl, Alkinyl, Arylalkyl, Aryl, Hetarylalkyl oder Hetaryl oder für eine der Gruppen -COR³, -CO₂R³, -CN, -CONR³R⁴, -SO₂R³ oder -P(O)(OR³)OR⁴ steht, worin
R³ und R⁴ unabhängig voneinander für Wasserstoff oder für jeweils gegebenenfalls substituiertes Alkyl, Alkenyl, Arylalkyl, Aryl, Hetarylalkyl oder Hetaryl stehen oder
R³ und R⁴ gemeinsam für eine gegebenenfalls substituierte Alkylen-Gruppe stehen, in welcher gegebenenfalls eine oder mehrere Methylengruppen durch die gleiche Anzahl Heteroatome ersetzt ist,
D für Wasserstoff oder für einen gegebenenfalls substituierten Rest aus der Reihe Alkyl, Alkenyl, Alkinyl, Alkoxyalkyl, Polyalkoxyalkyl, Alkylthioalkyl, gesättigtes oder ungesättigtes Cycloalkyl, gesättigtes oder ungesättigtes Heterocyclyl, Arylalkyl, Aryl, Hetarylalkyl und Hetaryl steht oder
A und D gemeinsam für eine jeweils gegebenenfalls substituierte Alkylen- oder Alkenylen-Gruppe stehen, in welcher jeweils gegebenenfalls eine oder mehrere Methylengruppen durch die gleiche Anzahl Heteroatome oder Heterogruppen ersetzt ist,
R¹ für Wasserstoff oder für gegebenenfalls durch Halogen substituiertes Alkyl steht und
R² für jeweils gegebenenfalls durch Halogen substituiertes Alkyl, Alkenyl oder Alkinyl steht.

2. Verbindungen der Formel (I) gemäß Anspruch 1, in welcher
X für Fluor, Chlor, Brom, Iod, Nitro, Cyano, C₁-C₈-Alkyl, C₂-C₆-Alkenyl, C₁-C₆-Alkoxy, C₂-C₆-Alkenyloxy, C₁-C₆-Alkylthio, C₁-C₆-Alkylsulfinyl, C₁-C₆-Alkylsulfonyl, für jeweils durch Fluor, Chlor oder Brom substituiertes C₁-C₆-Alkyl, C₂-C₆-Alkenyl, C₁-C₆-Alkoxy oder C₂-C₆-Alkenyloxy oder für jeweils gegebenenfalls durch Fluor, Chlor, Brom, Iod, Nitro, Cyano oder durch jeweils gegebenenfalls durch Fluor, Chlor oder Brom substituiertes C₁-C₆-Alkyl oder C₁-C₆-Alkoxy substituiertes Phenyl, Phenoxy, Phenylthio, Benzyloxy oder Benzylthio steht,
Y für Wasserstoff, Fluor, Chlor, Brom, Iod, Nitro, Cyano, C₁-C₈-Alkyl, C₂-C₆-Alkenyl, C₁-C₆-Alkoxy, C₂-C₆-Alkenyloxy, C₁-C₆-Alkylthio, C₁-C₆-Alkylsulfinyl, C₁-C₆-Alkylsulfonyl oder für jeweils durch Fluor, Chlor oder Brom substituiertes C₁-C₆-Alkyl, C₂-C₆-Alkenyl, C₁-C₆-Alkoxy oder C₂-C₆-Alkenyloxy steht,
Z für Fluor, Chlor, Brom, Iod, Nitro, Cyano, C₁-C₈-Alkyl, C₂-C₆-Alkenyl, C₁-C₆-Alkoxy, C₂-C₆-Alkenyloxy oder jeweils durch Fluor, Chlor oder Brom substituiertes C₁-C₆-Alkyl, C₂-C₆-Alkenyl, C₁-C₆-Alkoxy oder C₂-C₆-Alkenyloxy steht,
n für eine der Zahlen 0, 1, 2 oder 3 steht,
A für Wasserstoff, Fluor, Chlor, Brom oder Iod, für einen gegebenenfalls durch Fluor, Chlor, Brom oder Iod substituierten Rest aus der Reihe C₁-C₁₀-Alkyl, C₃-C₁₀-Cycloalkyl, C₃-C₁₀-Alkenyl und C₃-C₁₀-Alkinyl, für einen gegebenenfalls durch Fluor, Chlor, Brom, Iod, Nitro, C₁-C₈-Alkyl, C₁-C₈-Alkoxy, C₁-C₈-Alkylthio, C₁-C₈-Halogenalkyl, C₁-C₈-Halogenalkoxy oder Cyano substituierten Rest aus der Reihe Phenyl, Naphthyl, 5- oder 6-gliedriges Hetaryl mit ein bis drei Heteroatomen aus der Reihe Sauerstoff, Schwefel und Stickstoff, Phenyl-C₁-C₆-alkyl und 5- oder 6-gliedriges Hetaryl-C₁-C₆-alkyl mit ein bis drei Heteroatomen aus der Reihe Sauerstoff, Schwefel und Stickstoff oder für eine der Gruppen -COR³, -CO₂R³, -CN, -CONR³R⁴, -SO₂R³ oder -P(O)(OR³)OR⁴ steht, worin
R³ und R⁴ unabhängig voneinander für Wasserstoff, für jeweils gegebenenfalls durch Fluor, Chlor, Brom oder Iod substituiertes C₁-C₁₀-Alkyl oder C₃-C₁₀-Alkenyl oder für einen gegebenenfalls durch Fluor, Chlor, Brom, Iod, Nitro, C₁-C₈-Alkyl, C₁-C₈-Alkoxy, C₁-C₈-Alkylthio, C₁-C₈-Halogenalkyl, C₁-C₈-Halogenalkoxy oder Cyano substituierten Rest aus der Reihe Phenyl, Naphthyl, 5- oder 6-gliedriges Hetaryl mit ein bis drei Heteroatomen aus der Reihe Sauerstoff, Schwefel und Stickstoff, Phenyl-C₁-C₆-alkyl und 5- oder 6-gliedriges Hetaryl-C₁-C₆-alkyl mit ein bis drei Heteroatomen aus der Reihe Sauerstoff, Schwefel und Stickstoff stehen oder
R³ und R⁴ gemeinsam für eine C₂-C₇-Alkylen-Gruppe stehen, in welcher gegebenenfalls eine nicht endständige Methylengruppe durch Sauerstoff, Schwefel, NH oder N-C₁-C₄-Alkyl ersetzt ist,
D für Wasserstoff, für jeweils gegebenenfalls durch Fluor, Chlor, Brom oder Iod substituiertes C₁-C₁₂-Alkyl, C₃-C₈-Alkenyl, C₃-C₈-Alkinyl, C₁-C₁₀-Alkoxy-C₂-C₈-alkyl, Poly-C₁-C₈-alkoxy-C₂-C₈-alkyl oder C₁-C₁₀-Alkylthio-C₂-C₈-alkyl, für durch Cyano, C₁-C₈-Alkyloxycarbonyl oder C₁-C₈-Alkylcarbonyloxy substituiertes C₁-C₁₂-Alkyl, für gegebenenfalls durch Halogen, C₁-C₄-Alkyl, C₁-C₄-Alkoxy oder C₁-C₄-Halogenalkyl substituiertes C₃-C₈-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Hetaryl mit 5 bis 6 Ringatomen und ein bis drei Heteroatomen aus der Reihe Sauerstoff, Schwefel und Stickstoff, Phenyl-C₁-C₆-alkyl oder Hetaryl-C₁-C₆-alkyl mit 5 bis 6 Ringatomen und ein bis drei Heteroatomen aus der Reihe Sauerstoff, Schwefel und Stickstoff steht oder
A und D gemeinsam für eine jeweils gegebenenfalls durch C₁-C₈-Alkyl, Fluor, Chlor, Brom oder Iod substituierte C₂-C₇-Alkylen- oder C₂-C₇-Alkenylen-Gruppe stehen, worin gegebenenfalls zwei Kohlenstoffatome durch eine C₁-C₂-Alkylengruppe verbunden sind und in welcher gegebenenfalls eine Methylengruppe durch Sauerstoff, Schwefel, NH, N-C₁-C₄-Alkyl oder die Gruppe -O-CO- ersetzt ist,
R¹ für Wasserstoff oder für gegebenenfalls durch Fluor, Chlor, Brom oder lod substituiertes C₁-C₈-Alkyl steht und
R² für jeweils gegebenenfalls durch Fluor, Chlor, Brom oder Iod substituiertes C₁-C₁₀-Alkyl, C₃-C₁₀-Alkenyl oder C₃-C₁₀-Alkinyl steht.

3. Verbindungen der Formel (I) gemäß Anspruch 1, in welcher
X für Fluor, Chlor, Brom, Nitro, Cyano, C₁-C₆-Alkyl, C₂-C₄-Alkenyl, C₁-C₄-Alkoxy, C₂-C₄-Alkenyloxy, C₁-C₄-Alkylthio, C₁-C₄-Alkylsulfinyl, C₁-C₄-Alkylsulfonyl, für jeweils durch Fluor oder Chlor substituiertes C₁-C₄-Alkyl, C₂-C₄-Alkenyl, C₁-C₄-Alkoxy oder C₂-C₄-Alkenyloxy oder für jeweils gegebenenfalls durch Fluor, Chlor, Brom, Nitro, Cyano oder durch jeweils gegebenenfalls durch Fluor oder Chlor substituiertes C₁-C₄-Alkyl oder C₁-C₄-Alkoxy substituiertes Phenyl, Phenoxy, Phenylthio, Benzyloxy oder Benzylthio steht,
Y für Wasserstoff, Fluor, Chlor, Brom, Nitro, Cyano, C₁-C₆-Alkyl, C₂-C₄-Alkenyl, C₁-C₄-Alkoxy, C₂-C₄-Alkenyloxy, C₁-C₄-Alkylthio, C₁-C₄-Alkylsulfinyl, C₁-C₄-Alkylsulfonyl oder für jeweils durch Fluor oder Chlor substituiertes C₁-C₄-Alkyl oder C₁-C₄-Alkoxy steht,
Z für Fluor, Chlor, Brom, Nitro, Cyano, C₁-C₆-Alkyl, C₁-C₄-Alkoxy, C₂-C₄-Alkenyloxy oder jeweils durch Fluor oder Chlor substituiertes C₁-C₄-Alkyl oder C₁-C₄-Alkoxy steht,
n für eine der Zahlen 0, 1 oder 2 steht,
A für Wasserstoff, Fluor, Chlor oder Brom, für einen gegebenenfalls durch Fluor, Chlor oder Brom substituierten Rest aus der Reihe C₁-C₈-Alkyl, C₃-C₈-Cycloalkyl, C₃-C₈-Alkenyl und C₃-C₈-Alkinyl, für einen gegebenenfalls durch Fluor, Chlor, Brom, Nitro, C₁-C₄-Alkyl, C₁-C₄-Alkoxy, C₁-C₄-Alkylthio, C₁-C₄-Halogenalkyl, C₁-C₄-Halogenalkoxy oder Cyano substituierten Rest aus der Reihe Phenyl, Naphthyl, Furanyl, Thienyl, Pyridyl, Phenyl-C₁-C₄-alkyl, Furanyl-C₁-C₄-alkyl, Thienyl-C₁-C₄-alkyl und Pyridyl-C₁-C₄-alkyl oder für eine der Gruppen -COR³, -CO₂R³, -CN, -CONR³R⁴, -SO₂R³ oder -P(O)(OR³)OR⁴ steht, worin
R³ und R⁴ unabhängig voneinander für Wasserstoff, für jeweils gegebenenfalls durch Fluor, Chlor oder Brom substituiertes C₁-C₈-Alkyl oder C₃-C₈-Alkenyl oder für einen gegebenenfalls durch Fluor, Chlor, Brom, Nitro, C₁-C₄-Alkyl, C₁-C₄-Alkoxy, C₁-C₄-Alkylthio, C₁-C₄-Halogenalkyl, C₁-C₄-Halogenalkoxy oder Cyano substituierten Rest aus der Reihe Phenyl, Furanyl, Thienyl, Pyridyl, Phenyl-C₁-C₄-alkyl, Furanyl-C₁-C₄-alkyl, Thienyl-C₁-C₄-alkyl und Pyridyl-C₁-C₄-alkyl stehen oder
R³ und R⁴ gemeinsam für eine C₂-C₆-Alkylen-Gruppe stehen, in welcher gegebenenfalls eine nicht endständige Methylengruppe durch Sauerstoff, Schwefel, NH oder N-C₁-C₄-Alkyl ersetzt ist,
D für Wasserstoff, für jeweils gegebenenfalls durch Fluor oder Chlor substituiertes C₁-C₁₀-Alkyl, C₃-C₆-Alkenyl, C₃-C₆-Alkinyl, C₁-C₈-Alkoxy-C₂-C₆-alkyl, Poly-C₁-C₆-alkoxy-C₂-C₆-alkyl oder C₁-C₈-Alkylthio-C₂-C₆-alkyl, für durch Cyano, C₁-C₆-Alkoxycarbonyl oder C₁-C₆-Alkylcarbonyloxy substituiertes C₁-C₈-Alkyl, für gegebenenfalls durch Fluor, Chlor, C₁-C₄-Alkyl, C₁-C₄-Alkoxy oder C₁-C₂-Halogenalkyl substituiertes C₃-C₇-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, C₁-C₄-Alkyl, C₁-C₄-Halogenalkyl, C₁-C₄-Alkoxy, C₁-C₄-Halogenalkoxy, Cyano oder Nitro substituiertes Phenyl, Furanyl, Imidazolyl, Pyridyl, Thiazolyl, Pyrazolyl, Pyrimidyl, Pyridazyl, Pyrazinyl, Pyrrolyl, Thienyl, Triazolyl oder Phenyl-C₁-C₄-alkyl steht oder
A und D gemeinsam für eine jeweils gegebenenfalls durch C₁-C₄-Alkyl, Fluor, Chlor oder Brom substituierte C₂-C₆-Alkylen- oder C₂-C₆-Alkenylen-Gruppe stehen, worin gegebenenfalls zwei Kohlenstoffatome durch eine C₁-C₂-Alkylengruppe verbunden sind und in welcher gegebenenfalls eine Methylengruppe durch Sauerstoff, Schwefel, NH, N-C₁-C₄-Alkyl oder die Gruppe -O-CO- ersetzt ist,
R¹ für Wasserstoff oder für gegebenenfalls durch Fluor, Chlor oder Brom substituiertes C₁-C₄-Alkyl steht und
R² für jeweils gegebenenfalls durch Fluor, Chlor oder Brom substituiertes C₁-C₈-Alkyl, C₃-C₈-Alkenyl oder C₃-C₈-Alkinyl steht.

4. Verbindungen der Formel (I) gemäß Anspruch 1, in welcher
X für Fluor, Chlor, Brom, Nitro, Cyano, Methyl, Ethyl, n- oder i-Propyl, n-, s-, i- oder t-Butyl, Vinyl, Allyl, Methallyl, Methoxy, Ethoxy, n- oder i-Propoxy, Allyloxy, Methallyloxy, Trifluormethyl, Difluormethoxy, Trifluormethoxy, Trifluorethoxy, Methylthio, Methylsulfinyl oder Methylsulfonyl steht,
Y für Wasserstoff, Fluor, Chlor, Brom, Nitro, Cyano, Methyl, Ethyl, n- oder i-Propyl, n-, s-, i- oder t-Butyl, Methoxy, Ethoxy, n- oder i-Propoxy, Allyloxy, Methallyloxy, Trifluormethyl, Difluormethoxy, Trifluormethoxy, Trifluorethoxy, Methylthio, Methylsulfinyl oder Methylsulfonyl steht,
Z für Fluor, Chlor, Brom, Nitro, Cyano, Methyl, Ethyl, n- oder i-Propyl, n-, s-, i- oder t-Butyl, Methoxy, Ethoxy, n- oder i-Propoxy, Allyloxy, Methallyloxy, Trifluormethyl, Difluormethoxy, Trifluormethoxy oder Trifluorethoxy steht,
n für eine der Zahlen 0 oder 1 steht,
A für Wasserstoff, Fluor, Chlor oder Brom, für einen gegebenenfalls durch Fluor oder Chlor substituierten Rest aus der Reihe C₁-C₆-Alkyl, C₃-C₆-Cycloalkyl, C₃-C₆-Alkenyl und C₃-C₆-Alkinyl, für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl, Furanyl, Pyridyl, Thienyl oder Benzyl oder für eine der Gruppen -COR³, -CO₂R³, -CN, -CONR³R⁴, -SO₂R³ oder -P(O)(OR³)OR⁴ steht, worin
R³ und R⁴ unabhängig voneinander für Wasserstoff, für jeweils gegebenenfalls durch Fluor oder Chlor substituiertes C₁-C₆-Alkyl oder C₃-C₆-Alkenyl oder für gegebenenfalls durch Fluor, Chlor, Brom, Nitro, C₁-C₄-Alkyl, C₁-C₄-Alkoxy, C₁-C₄-Alkylthio, C₁-C₄-Halogenalkyl, C₁-C₄-Halogenalkoxy oder Cyano substituiertes Phenyl stehen oder
R³ und R⁴ gemeinsam für eine der Gruppen -(CH₂)₃-, -(CH₂)₄-, -(CH₂)₅-, -CH₂-CH(CH₃)(CH₂)₂-, -CH₂O-(CH₂)₂- und -CH₂S-(CH₂)₂- stehen,
D für Wasserstoff, für jeweils gegebenenfalls durch Fluor oder Chlor substituiertes C₁-C₈-Alkyl, C₃-C₄-Alkenyl, C₃-C₄-Alkinyl, C₁-C₆-Alkoxy-C₂-C₄-alkyl, Poly-C₁-C₄-alkoxy-C₂-C₄-alkyl oder C₁-C₄-Alkylthio-C₂-C₄-alkyl oder für gegebenenfalls durch Fluor, Chlor, Methyl oder Ethyl substituiertes C₃-C₆-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl, Furanyl, Pyridyl, Thienyl oder Benzyl steht oder
A und D gemeinsam für eine der Gruppen -(CH₂)₃-, -(CH₂)₄-, -(CH₂)₅-, -CH₂-CH(CH₃)(CH₂)₂-, -CH₂O-(CH₂)₂-, -CH₂S-(CH₂)₂-oder 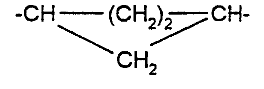 stehen,
R¹ für Wasserstoff oder für gegebenenfalls durch Fluor oder Chlor substituiertes Methyl oder Ethyl steht und
R² für jeweils gegebenenfalls durch Fluor oder Chlor substituiertes Methyl, Ethyl, n- oder i-Propyl, n-, i-, s- oder t-Butyl, Allyl, Methallyl, Butenyl, Propargyl oder Butinyl steht.

5. Verfahren zur Herstellung von Verbindungen der Formel (I) gemäß Anspruch 1, **dadurch gekennzeichnet, daß** man Verbindungen der Formel (II) 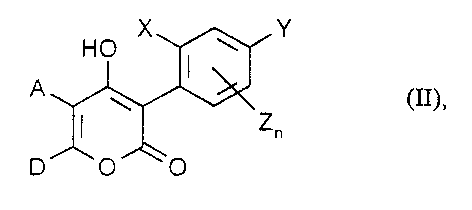 in welcher
A, D, X, Y, Z und n die in Anspruch 1 angegebenen Bedeutungen haben, mit Verbindungen der Formel (III) 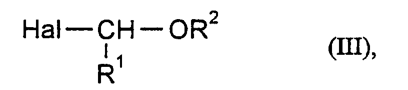 in welcher
R¹ und R² die in Anspruch 1 angegebenen Bedeutungen haben und
Hal für Halogen steht,
gegebenenfalls in Gegenwart eines Verdünnungsmittels und gegebenenfalls in Gegenwart eines Reaktionshilfsmittels umsetzt.

6. Schädlingsbekämpfungsmittel, **gekennzeichnet durch** einen Gehalt an mindestens einer Verbindung der Formel (I) gemäß Anspruch 1.

7. Verwendung von Verbindungen der Formel (I) gemäß Anspruch 1 zur Bekämpfung von Schädlingen.

8. Verfahren zur Bekämpfung von Schädlingen, **dadurch gekennzeichnet, daß** man Verbindungen der Formel (I) gemäß Anspruch 1 auf Schädlinge und/oder ihren Lebensraum einwirken läßt.

9. Verfahren zur Herstellung von Schädlingsbekämpfungsmitteln, **dadurch gekennzeichnet, daß** man Verbindungen der Formel (I) gemäß Anspruch 1 mit Streckmitteln und/oder oberflächenaktiven Mitteln vermischt.

10. Verwendung von Verbindungen der Formel (I) gemäß Anspruch 1 zur Herstellung von Schädlingsbekämpfungsmitteln.

## Claims

1. Compounds of the formula (I) 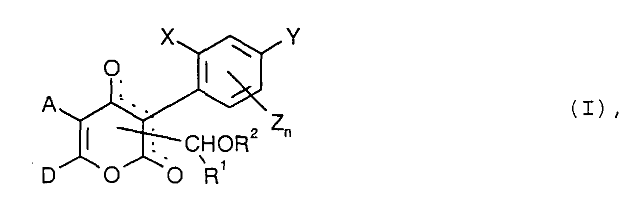 in which
X represents halogen, nitro, cyano, alkyl, alkenyl, alkoxy, alkenyloxy, alkylthio, alkylsulfinyl, alkylsulfonyl, halogenoalkyl, halogenoalkenyl, halogenoalkoxy, halogenoalkenyloxy or in each case unsubstituted or substituted phenyl, phenoxy, phenylthio, benzyloxy or benzylthio,
Y represents hydrogen, halogen, nitro, cyano, alkyl, alkenyl, alkoxy, alkenyloxy, alkylthio, alkylsulfinyl, alkylsulfonyl, halogenoalkyl, halogenoalkenyl, halogenoalkoxy or halogenoalkenyloxy,
Z represents halogen, nitro, cyano, alkyl, alkenyl, alkoxy, alkenyloxy, halogenoalkyl, halogenoalkenyl, halogenoalkoxy or halogenoalkenyloxy,
n represents one of the numbers 0 ; 1, 2 or 3,
A represents hydrogen, halogen; an unsubstituted or substituted radical selected from the group comprising alkyl, cycloalkyl, alkenyl, alkinyl, arylalkyl, aryl, hetarylalkyl or hetaryl; or one of the groups -COR³, -CO₂R³, -CN, -CONR³R⁴, -SO₂R³ or -P(O) (OR³) OR⁴, in which
R³ and R⁴ independently of one another represent hydrogen or unsubstituted or substituted alkyl, alkenyl, arylalkyl, aryl, hetarylalkyl or hetaryl or
R³ and R⁴ together represent an unsubstituted or substituted alkylene group, in which one or more methylene groups is or are optionally replaced by the same number of hetero atoms,
D represents hydrogen or an unsubstituted or substituted radical selected from the group comprising alkyl, alkenyl, alkinyl, alkoxyalkyl, polyalkoxyalkyl, alkylthioalkyl, saturated or unsaturated cycloalkyl, saturated or unsaturated heterocyclyl, arylalkyl, aryl, hetarylalkyl and hetaryl or
A and D together represent in each case an unsubstituted or substituted alkylene or alkenylene group, in each of which one or more methylene groups is or are optionally replaced by the same number of hetero atoms or hetero groups,
R¹ represents hydrogen or alkyl which is optionally substituted by halogen and
R² represents alkyl, alkenyl or alkinyl, each of which is optionally substituted by halogen.

2. Compounds of the formula (I) according to Claim 1, in which
X represents fluorine, chlorine, bromine, iodine, nitro, cyano, C₁-C₈-alkyl, C₂-C₆-alkenyl, C₁-C₆-alkoxy, C₂-C₆-alkenyloxy, C₁-C₆-alkylthio, C₁-C₆-alkylsulfinyl, C₁-C₆-alkylsulfonyl; C₁-C₆-alkyl, C₂-C₆-alkenyl, C₁-C₆-alkoxy or C₂-C₆-alkenyloxy, each of which is substituted by fluorine, chlorine or bromine; or phenyl, phenoxy, phenylthio, benzyloxy or benzylthio, each of which is optionally substituted by fluorine, chlorine, bromine, iodine, nitro, cyano or by C₁-C₆-alkyl or C₁-C₆-alkoxy, each of which is optionally substituted by fluorine, chlorine or bromine,
Y represents hydrogen, fluorine, chlorine, bromine, iodine, nitro, cyano, C₁-C₈-alkyl, C₂-C₆-alkenyl, C₁-C₆-alkoxy, C₂-C₆-alkenyloxy, C₁-C₆-alkylthio, C₁-C₆-alkylsulfinyl, C₁-C₆-alkylsulfonyl; or C₁-C₆-alkyl, C₂-C₆-alkenyl, C₁-C₆-alkoxy or C₂-C₆-alkenyloxy, each of which is substituted by fluorine, chlorine or bromine,
z represents fluorine, chlorine, bromine, iodine, nitro, cyano, C₁-C₈-alkyl, C₂-C₆-alkenyl, C₁-C₆-alkoxy, C₂-C₆-alkenyloxy or C₁-C₆-alkyl, C₂-C₆-alkenyl, C₁-C₆-alkoxy or C₂-C₆-alkenyloxy each of which is substituted by fluorine, chlorine or bromine,
n represents one of the numbers 0, 1, 2 or 3,
A represents hydrogen, fluorine, chlorine, bromine or iodine; a radical, selected from the group consisting of C₁-C₁₀-alkyl, C₃-C₁₀-cycloalkyl, C₃-C₁₀-alkenyl and C₃-C₁₀-alkinyl, which radical is optionally substituted by fluorine, chlorine, bromine or iodine; a radical selected from the group consisting of phenyl, naphthyl, 5- or 6-membered hetaryl having one to three hetero atoms selected from the group consisting of oxygen, sulfur and nitrogen, phenyl-C₁-C₆-alkyl and 5- or 6-membered hetaryl-C₁-C₆-alkyl having one to three hetero atoms selected from the group consisting of oxygen, sulfur and nitrogen, which radical is optionally substituted by fluorine, chlorine, bromine, iodine, nitro, C₁-C₈-alkyl, C₁-C₈-alkoxy, C₁-C₈-alkylthio, C₁-C₈-halogenoalkyl, C₁-C₈-halogenoalkoxy or cyano; or one of the groups -COR³, -CO₂R³, -CN, -CONR³R⁴, -SO₂R³ or -P(O)(OR³)OR⁴, in which
R³ and R⁴ independently of one another represent hydrogen; C₁-C₁₀-alkyl or C₃-C₁₀-alkenyl, each of which is optionally substituted by fluorine, chlorine, bromine or iodine; or a radical selected from the group consisting of phenyl, naphthyl, 5- or 6-membered hetaryl having one to three hetero atoms selected from the group consisting of oxygen, sulfur and nitrogen, phenyl-C₁-C₆-alkyl and 5- or 6-membered hetaryl-C₁-C₆-alkyl having one to three hetero atoms selected from the group consisting of oxygen, sulfur and nitrogen which radical is optionally substituted by fluorine, chlorine, bromine, iodine, nitro, C₁-C₈-alkyl, C₁-C₈-alkoxy, C₁-C₈-alkylthio, C₁-C₈-halogenoalkyl, C₁-C₈-halogenoalkoxy or cyano, or
R³ and R⁴ together represent a C₂-C₇-alkylene group in which one non-terminal methylene group is optionally replaced by oxygen, sulfur, NH or N-C₁-C₄-alkyl,
D represents hydrogen; C₁-C₁₂-alkyl, C₃-C₈-alkenyl, C₃-C₈-alkinyl, C₁-C₁₀-alkoxy-C₂-C₈-alkyl, poly-C₁-C₈-alkoxy-C₂-C₈-alkyl or C₁-C₁₀-alkylthio-C₂-C₈-alkyl, each of which is optionally substituted by fluorine, chlorine, bromine or iodine; cyano-, C₁-C₈-alkyloxycarbonyl- or C₁-C₈-alkylcarbonyloxysubstituted C₁-C₁₂-alkyl; C₃-C₈-cycloalkyl, which is optionally substituted by halogen, C₁-C₄-alkyl, C₁-C₄-alkoxy or C₁-C₄-halogenoalkyl and in which one or two not directly adjacent methylene groups are optionally replaced by oxygen and/or sulfur; or phenyl, hetaryl having 5 or 6 ring atoms and one to three hetero atoms selected from the group consisting of oxygen, sulfur and nitrogen, phenyl-C₁-C₆-alkyl or hetaryl-C₁-C₆-alkyl having 5 or 6 ring atoms and one to three hetero atoms selected from the group consisting of oxygen, sulfur and nitrogen, each of which is optionally substituted by halogen, C₁-C₆-alkyl, C₁-C₆-halogenoalkyl, C₁-C₆-alkoxy, C₁-C₆-halogenoalkoxy, cyano or nitro, or
A and D together represent a C₂-C₇-alkylene or C₂-C₇-alkenylene group, each of which is optionally substituted by C₁-C₈-alkyl, fluorine, chlorine, bromine or iodine and in which two carbon atoms are optionally joined by a C₁-C₂-alkylene group and in which a methylene group is optionally replaced by oxygen, sulfur, NH, N-C₁-C₄-alkyl or the group -O-CO-,
R¹ represents hydrogen or C₁-C₈-alkyl, which is optionally substituted by fluorine, chlorine, bromine or iodine and
R² represents C₁-C₁₀-alkyl, C₃-C₁₀-alkenyl or C₃-C₁₀-alkinyl, each of which is optionally substituted by fluorine, chlorine, bromine or iodine.

3. Compounds of the formula (I) according to Claim 1, in which
X represents fluorine, chlorine, bromine, nitro, cyano, C₁-C₆-alkyl, C₂-C₄-alkenyl, C₁-C₄-alkoxy, C₂-C₄-alkenyloxy, C₁-C₄-alkylthio, C₁-C₄-alkylsulfinyl, C₁-C₄-alkylsulfonyl; C₁-C₄-alkyl, C₂-C₄-alkenyl, C₁-C₄-alkoxy or C₂-C₄-alkenyloxy, each of which is substituted by fluorine or chlorine; or phenyl, phenoxy, phenylthio, benzyloxy or benzylthio, each of which is optionally substituted by fluorine, chlorine, bromine, nitro or cyano or by C₁-C₄-alkyl or C₁-C₄-alkoxy, each of which is optionally substituted by fluorine or chlorine,
Y represents hydrogen, fluorine, chlorine, bromine, nitro, cyano, C₁-C₆-alkyl, C₂-C₄-alkenyl, C₁-C₄-alkoxy, C₂-C₄-alkenyloxy, C₁-C₄-alkylthio, C₁-C₄-alkylsulfinyl, C₁-C₄-alkylsulfonyl; or C₁-C₄-alkyl or C₁-C₄-alkoxy, each of which is substituted by fluorine or chlorine,
Z represents fluorine, chlorine, bromine, nitro, cyano, C₁-C₆-alkyl, C₁-C₄-alkoxy, C₂-C₄-alkenyloxy; or C₁-C₄-alkyl or C₁-C₄-alkoxy, each of which is substituted by fluorine or chlorine,
n represents one of the numbers 0, 1 or 2,
A represents hydrogen, fluorine, chlorine or bromine; a radical selected from the group consisting of C₁-C₈-alkyl, C₃-C₈-cycloalkyl, C₃-C₈-alkenyl and C₃-C₈-alkinyl, which radical is optionally substituted by fluorine, chlorine or bromine; a radical selected from the group consisting of phenyl, naphthyl, furanyl, thienyl, pyridyl, phenyl-C₁-C₄-alkyl, furanyl-C₁-C₄-alkyl, thienyl-C₁-C₄-alkyl and pyridyl-C₁-C₄-alkyl, which radical is optionally substituted by fluorine, chlorine, bromine, nitro, C₁-C₄-alkyl, C₁-C₄-alkoxy, C₁-C₄-alkylthio, C₁-C₄-halogenoalkyl, C₁-C₄-halogenoalkoxy or cyano; or one of the groups -COR³, -CO₂R³, -CN, -CONR³R⁴, -SO₂R³ or -P(O)(OR³)OR⁴, in which
R³ and R⁴ independently of one another represent hydrogen; C₁-C₈-alkyl or C₃-C₈-alkenyl, each of which is optionally substituted by fluorine, chlorine or bromine; or a radical selected from the group consisting of phenyl, furanyl, thienyl, pyridyl, phenyl-C₁-C₄-alkyl, furanyl-C₁-C₄-alkyl, thienyl-C₁-C₄-alkyl and pyridyl-C₁-C₄-alkyl, which radical is optionally substituted by fluorine, chlorine, bromine, nitro, C₁-C₄-alkyl, C₁-C₄-alkoxy, C₁-C₄-alkylthio, C₁-C₄-halogenoalkyl, C₁-C₄-halogenoalkoxy or cyano, or
R³ and R⁴ together represent a C₂-C₆-alkylene group, in which a non-terminal methylene group is optionally replaced by oxygen, sulfur NH or N-C₁-C₄-alkyl,
D represents hydrogen; C₁-C₁₀-alkyl, C₃-C₆-alkenyl, C₃-C₆-alkinyl, C₁-C₈-alkoxy-C₂-C₆-alkyl, poly-C₁-C₆-alkoxy-C₂-C₆-alkyl or C₁-C₈-alkylthio-C₂-C₆-alkyl, each of which is optionally substituted by fluorine or chlorine; C₁-C₈-alkyl which is substituted by cyano, C₁-C₆-alkoxycarbonyl or C₁-C₆-alkylcarbonyloxy; C₃-C₇-cycloalkyl which is optionally substituted by fluorine, chlorine, C₁-C₄-alkyl, C₁-C₄-alkoxy or C₁-C₂-halogenoalkyl, and in which one or two non-directly adjacent methylene groups are optionally replaced by oxygen and/or sulfur; or phenyl, furanyl, imidazolyl, pyridyl, thiazolyl, pyrazolyl, pyrimidyl, pyridazyl, pyrazinyl, pyrrolyl, thienyl, triazolyl or phenyl-C₁-C₄-alkyl, each of which is optionally substituted by fluorine, chlorine, bromine, C₁-C₄-alkyl, C₁-C₄-halogenoalkyl, C₁-C₄-alkoxy, C₁-C₄-halogenoalkoxy, cyano or nitro, or
A and D together represent a C₂-C₆-alkylene or C₂-C₆-alkenylene group, each of which is optionally substituted by C₁-C₄-alkyl, fluorine, chlorine or bromine, in which two carbon atoms are optionally connected by a C₁-C₂-alkylene group and in which a methylene group is optionally replaced by oxygen, sulfur, NH, N-C₁-C₄-alkyl or the group -O-CO-,
R¹ represents hydrogen or C₁-C₄-alkyl which is optionally substituted by fluorine, chlorine or bromine and
R² represents C₁-C₈-alkyl, C₃-C₈-alkenyl or C₃-C₈-alkinyl, each of which is optionally substituted by fluorine, chlorine or bromine.

4. Compounds of the formula (I) according to Claim 1, in which
X represents fluorine, chlorine, bromine, nitro, cyano, methyl, ethyl, n- or i-propyl, n-, s-, i-or t-butyl, vinyl, allyl, methallyl, methoxy, ethoxy, n- or i-propoxy, allyloxy, methallyloxy, trifluoromethyl, difluoromethoxy, trifluoromethoxy, trifluoroethoxy, methylthio, methylsulfinyl or methylsulfonyl,
Y represents hydrogen, fluorine, chlorine, bromine, nitro, cyano, methyl, ethyl, n- or i-propyl, n-, s-, i- or t-butyl, methoxy, ethoxy, n- or i-propoxy, allyloxy, methallyloxy, trifluoromethyl, difluoromethoxy, trifluoromethoxy, tri-fluoroethoxy, methylthio, methylsulfinyl or methylsulfonyl,
Z represents fluorine, chlorine, bromine, nitro, cyano, methyl, ethyl, n- or i-propyl, n-, s-, i-or t-butyl, methoxy, ethoxy, n- or i-propoxy, allyloxy, methallyloxy, trifluoromethyl, difluoromethoxy, trifluoromethoxy or trifluoroethoxy,
n represents either of the numbers 0 or 1,
A represents hydrogen, fluorine, chlorine or bromine; a radical selected from the group consisting of C₁-C₆-alkyl, C₃-C₆-cycloalkyl, C₃-C₆-alkenyl and C₃-C₆-alkinyl, which radical is optionally substituted by fluorine or chlorine; phenyl, furanyl, pyridyl, thienyl or benzyl, each of which is optionally substituted by fluorine, chlorine, bromine, methyl, ethyl, n-propyl, isopropyl, methoxy, ethoxy, trifluoromethyl, trifluoromethoxy, cyano or nitro; or one of the groups -COR³, -CO₂R³, -CN, -CONR³R⁴, -SO₂R³ or -P(O)(OR³)OR⁴, in which
R³ and R⁴ independently of one another represent hydrogen; C₁-C₆-alkyl or C₃-C₆-alkenyl, each of which is optionally substituted by fluorine or chlorine; or phenyl which is optionally substituted by fluorine, chlorine, bromine, nitro, C₁-C₄-alkyl, C₁-C₄-alkoxy, C₁-C₄-alkylthio, C₁-C₄-halogenoalkyl, C₁-C₄-halogenoalkoxy or cyano, or
R³ and R⁴ together represent one of the groups -(CH₂)₃-, -(CH₂)₄-, -(CH₂)₅-, -CH₂-CH(CH₃(CH₂)₂-, CH₂O-(CH₂)₂- and -CH₂S- (CH₂)₂-,
D represents hydrogen; C₁-C₈-alkyl, C₃-C₄-alkenyl, C₃-C₄-alkinyl, C₁-C₆-alkoxy-C₂-C₄-alkyl, poly-C₁-C₄-alkoxy-C₂-C₄-alkyl or C₁-C₄-alkylthio-C₂-C₄-alkyl, each of which is optionally substituted by fluorine or chlorine; or C₃-C₆-cycloalkyl which is optionally substituted by fluorine, chlorine, methyl or ethyl and in which one or two non-directly adjacent methylene groups are optionally replaced by oxygen and/or sulfur; or phenyl, furanyl, pyridyl, thienyl or benzyl, each of which is optionally substituted by fluorine, chlorine, bromine, methyl, ethyl, n-propyl, isopropyl, methoxy, ethoxy, trifluoromethyl, trifluoromethoxy, cyano or nitro, or
A and D together represent one of the groups - (CH₂)₃, - (CH₂)₄, -(CH₂)₅-, -CH₂-CH(CH₃) (CH₂)₂-, -CH₂O-(CH₂)₂-, -CH₂S- (CH₂)₂- or 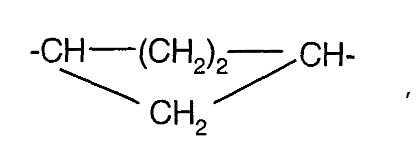
R¹ represents hydrogen; or methyl or ethyl, each of which is optionally substituted by fluorine or chlorine and
R² represents methyl, ethyl, n- or i-propyl, n-, i-, s- or t-butyl, allyl, methallyl, butenyl, propargyl or butinyl, each of which is optionally substituted by fluorine or chlorine.

5. Process for the preparation of compounds of the formula (I) according to Claim 1, **characterized in that** compounds of the formula (II) 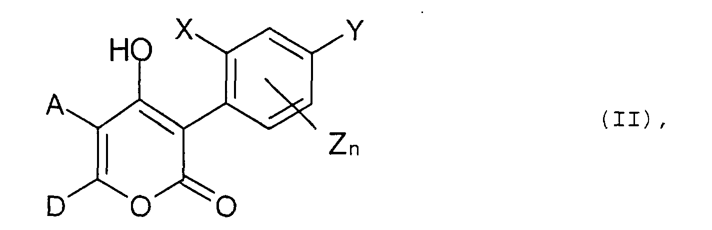 in which
A, D, X, Y, Z and n have the meanings specified in claim 1, are reacted with compounds of the formula (III) 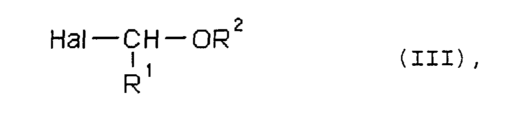
in which
R¹ and R² have the meanings specified in Claim 1 and
Hal represents halogen,
in the presence or absence of a diluent and in the presence or absence of a reaction auxiliary.

6. Pesticides, **characterized by** a content of at least one compound of the formula (I) according to Claim 1.

7. Use of compounds of the formula (I) according to Claim 1 for controlling pests.

8. Method of controlling pests, **characterized in that** compounds of the formula (I) according to Claim 1 are allowed to act on pests and/or their habitat.

9. Process for the preparation of pesticides, **characterized in that** compounds of the formula (I) according to Claim 1 are mixed with extenders and/or surfactants.

10. Use of compounds of the formula (I) according to Claim 1 for the preparation of pesticides.

## Revendications

1. Composés de formule (I) 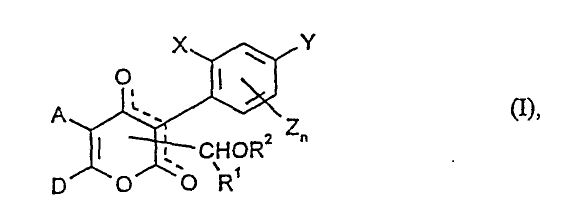 dans laquelle
X représente un halogène, un groupe nitro, cyano, alkyle, alcényle, alkoxy, alcényloxy, alkylthio, alkylsulfinyle, alkylsulfonyle, halogénalkyle, halogénalcényle, halogénalkoxy, halogénalcényloxy ou un groupe phényle, phénoxy, phénylthio, benzyloxy ou benzylthio dont chacun est éventuellement substitué,
Y représente l'hydrogène, un halogène, un groupe nitro, cyano, alkyle, alcényle, alkoxy, alcényloxy, alkylthio, alkylsulfinyle, alkylsulfonyle, halogénalkyle, halogénalcényle, halogénalkoxy ou halogénalcényloxy,
Z représente un halogène, un groupe nitro, cyano, alkyle, alcényle, alkoxy, alcényloxy, halogénalkyle, halogénalcényle, halogénalkoxy ou halogénalcényloxy,
n représente l'un des nombres 0, 1, 2 et 3,
A représente l'hydrogène, un halogène, un reste éventuellement substitué de la série alkyle, cycloalkyle, alcényle, alcynyle, arylalkyle, aryle, hétarylalkyle ou hétaryle ou l'un des groupes -COR³, -CO₂R³, -CN, - CONR³R⁴, -SO₂R³ ou -P(O) (OR³)OR⁴, où
R³ et R⁴ représentent, indépendamment l'un de l'autre, l'hydrogène ou un groupe alkyle, alcényle, arylalkyle, aryle, hétarylalkyle ou hétaryle dont chacun est éventuellement substitué,
R³ et R⁴ forment ensemble un groupe alkylène éventuellement substitué, dans lequel le cas échéant un ou plusieurs chaînons méthylène sont remplacés par le même nombre d'hétéro-atomes,
D représente l'hydrogène ou un reste éventuellement substitué de la série alkyle, alcényle, alcynyle, alkoxyalkyle, polyalkoxyalkyle, alkylthioalkyle, cycloalkyle saturé ou non saturé, hétérocyclyle saturé ou non saturé, arylalkyle, aryle, hétarylalkyle et hétaryle, ou bien
A et D forment ensemble un groupe alkylène ou un groupe alcénylène dont chacun est éventuellement substitué et dans chacun desquels le cas échéant un ou plusieurs chaînons méthylène sont remplacés par le même nombre d'hétéro-atomes ou d'hétéro-groupes,
R¹ représente l'hydrogène ou un groupe alkyle éventuellement substitué par un halogène et
R² représente un groupe alkyle, alcényle ou alcynyle dont chacun est éventuellement substitué par un halogène.

2. Composés de formule (I) suivant la revendication 1, dans laquelle
X représente le fluor, le chlore, le brome, l'iode, un groupe nitro, cyano, alkyle en C₁ à C₈, alcényle en C₂ à C₆, alkoxy en C₁ à C₆, alcényloxy en C₂ à C₆, alkylthio en C₁ à C₆, alkylsulfinyle en C₁ à C₆, alkylsulfonyle en C₁ à C₆, un groupe alkyle en C₁ à C₆, alcényle en C₂ à C₆, alkoxy en C₁ à C₆ ou alcényloxy en C₂ à C₆ dont chacun est substitué par du fluor, du chlore ou du brome, ou un groupe phényle, phénoxy, phénylthio, benzyloxy ou benzylthio dont chacun est éventuellement substitué par du fluor, du chlore, du brome, de l'iode, un radical nitro, cyano ou par un radical alkyle en C₁ à C₆ ou alkoxy en C₁ à C₆ dont chacun est éventuellement substitué par du fluor, du chlore ou du brome,
Y représente l'hydrogène, le fluor, le chlore, le brome, l'iode, un groupe nitro, cyano, alkyle en C₁ à C₈, alcényle en C₂ à C₆, alkoxy en C₁ à C₆, alcényloxy en C₂ à C₆, alkylthio en C₁ à C₆, alkylsulfinyle en C₁ à C₆, alkylsulfonyle en C₁ à C₆ ou un groupe alkyle en C₁ à C₆, alcényle en C₂ à C₆, alkoxy en C₁ à C₆ ou alcényloxy en C₂ à C₆ dont chacun est substitué par du fluor, du chlore ou du brome,
Z représente le fluor, le chlore, le brome, l'iode, un groupe nitro, cyano, alkyle en C₁ à C₈, alcényle en C₂ à C₆, alkoxy en C₁ à C₆, alcényloxy en C₂ à C₆, ou bien un groupe alkyle en C₁ à C₆, alcényle en C₂ à C₆, alkoxy en C₁ à C₆ ou alcényloxy en C₂ à C₆ dont chacun est substitué par du fluor, du chlore ou du brome,
n représente l'un des nombres 0, 1, 2 et 3,
A représente l'hydrogène, le fluor, le chlore, le brome ou l'iode, un reste, éventuellement substitué par du fluor, du chlore, du brome ou de l'iode, de la série alkyle en C₁ à C₁₀, cycloalkyle en C₃ à C₁₀, alcényle en C₃ à C₁₀ et alcynyle en C₃ à C₁₀, un reste portant éventuellement un substituant fluoro, chloro, bromo, iodo, nitro, alkyle en C₁ à C₈, alkoxy en C₁ à C₈, alkylthio en C₁ à C₈, halogénalkyle en C₁ à C₈, halogénalkoxy en C₁ à C₈ ou cyano, de la série phényle, naphtyle, hétaryle pentagonal ou hexagonal ayant 1 à 3 hétéro-atomes de la série oxygène, soufre et azote, phényl-(alkyle en C₁ à C₆) et (hétaryle pentagonal ou hexagonal)-(alkyle en C₁ à C₆) ayant 1 à 3 hétéro-atomes de la série oxygène, soufre et azote ou l'un des groupes -COR³, -CO₂R³, -CN, -CONR³R⁴, -SO₂R³ ou -P(O) (OR³)OR⁴, où
R³ et R⁴ représentent, indépendamment l'un de l'autre, l'hydrogène, un groupe alkyle en C₁ à C₁₀ ou alcényle en C₃ à C₁₀ éventuellement substitué par du fluor, du chlore, du brome ou de l'iode, ou un reste, éventuellement substitué par un radical fluoro, chloro, bromo, iodo, nitro, alkyle en C₁ à C₈, alkoxy en C₁ à C₈, alkylthio en C₁ à C₈, halogénalkyle en C₁ à C₈, halogénalkoxy en C₁ à C₈ ou cyano, de la série phényle, naphtyle, hétaryle pentagonal ou hexagonal ayant jusqu'à 3 hétéro-atomes de la série oxygène, soufre et azote, phényl-(alkyle en C₁ à C₆) et (hétaryle pentagonal ou hexagonal)-(alkyle en C₁ à C₆) ayant 1 à 3 hétéro-atomes de la série oxygène, soufre et azote, ou bien
R³ et R⁴ forment ensemble un groupe alkylène en C₂ à C₇ dans lequel, le cas échéant, un groupe méthylène non terminal est remplacé par de l'oxygène, du soufre, un groupe NH ou N-(alkyle en C₁ à C₄),
D représente l'hydrogène, un groupe alkyle en C₁ à C₁₂, alcényle en C₃ à C₈, alcynyle en C₃ à C₈, (alkoxy en C₁ à C₁₀)-(alkyle en C₂ à C₈), poly-(alkoxy en C₁ à C₈)-(alkyle en C₂ à C₈) ou (alkylthio en C₁ à C₁₀)-(alkyle en C₂ à C₈) dont chacun est éventuellement substitué par du fluor, du chlore, du brome ou de l'iode, un groupe alkyle en C₁ à C₁₂ éventuellement substitué par un radical cyano, (alkyloxy en C₁ à C₈)-carbonyle ou (alkyle en C₁ à C₈)-carbonyloxy, un groupe cycloalkyle en C₃ à C₈ éventuellement substitué par un halogène, un radical alkyle en C₁ à C₄, alkoxy en C₁ à C₄ ou halogénalkyle en C₁ à C₄, dans lequel un ou deux chaînons méthylène non contigus sont remplacés par de l'oxygène et/ou du soufre, ou un groupe phényle, hétaryle, pentagonal ou hexagonal, ayant 1 à 3 hétéro-atomes de la série oxygène, soufre et azote, phényl-(alkyle en C₁ à C₆) ou hétaryl- (alkyle en C₁ à C₆) pentagonal ou hexagonal ayant 1 à 3 hétéro-atomes de la série oxygène, soufre et azote, chacun étant éventuellement substitué par un radical halogéno, alkyle en C₁ à C₆, halogénalkyle en C₁ à C₆, alkoxy en C₁ à C₆, halogénalkoxy en C₁ à C₆, cyano ou nitro, ou bien
A et D forment ensemble un groupe alkylène en C₂ à C₇ ou alcénylène en C₂ à C₇ dont chacun est éventuellement substitué par un radical alkyle en C₁ à C₈, du fluor, du chlore, du brome ou de l'iode, dans lequel le cas échéant deux atomes de carbone sont liés par un groupe alkylène en C₁ ou C₂ et dans lequel, le cas échéant, un chaînon méthylène est remplacé par de l'oxygène, du soufre, un groupe NH, N-(alkyle en C₁ à C₄) ou le groupe -O-CO-,
R¹ représente l'hydrogène ou un groupe alkyle en C₁ à C₈, éventuellement substitué par du fluor, du chlore, du brome ou de l'iode, et
R² représente un groupe alkyle en C₁ à C₁₀, alcényle en C₃ à C₁₀ ou alcynyle en C₃ à C₁₀ dont chacun est éventuellement substitué par du fluor, du chlore, du brome ou de l'iode.

3. Composés de formule (I) suivant la revendication 1, dans laquelle
X représente le fluor, le chlore, le brome, un groupe nitro, cyano, alkyle en C₁ à C₆, alcényle en C₂ à C₄, alkoxy en C₁ à C₄, alcényloxy en C₂ à C₄, alkylthio en C₁ à C₄, alkylsulfinyle en C₁ à C₄, alkylsulfonyle en C₁ à C₄, un groupe alkyle en C₁ à C₄, alcényle en C₂ à C₄, alkoxy en C₁ à C₄ ou alcényloxy en C₂ à C₄ dont chacun est substitué par du fluor ou du chlore, ou un groupe phényle, phénoxy, phénylthio, benzyloxy ou benzylthio dont chacun est éventuellement substitué par du fluor, du chlore, du brome, un radical nitro, cyano ou par un radical alkyle en C₁ à C₄ ou alkoxy en C₁ à C₄ dont chacun est éventuellement substitué par du fluor ou du chlore,
Y représente l'hydrogène, le fluor, le chlore, le brome, un groupe nitro, cyano, alkyle en C₁ à C₆, alcényle en C₂ à C₄, alkoxy en C₁ à C₄, alcényloxy en C₂ à C₄, alkylthio en C₁ à C₄, alkylsulfinyle en C₁ à C₄, alkylsulfonyle en C₁ à C₄ ou un groupe alkyle en C₁ à C₄ ou alkoxy C₁ à C₄, dont chacun est substitué par du fluor ou du chlore,
Z représente le fluor, le chlore, le brome, un groupe nitro, cyano, alkyle en C₁ à C₆, alkoxy en C₁ à C₄, alcényloxy en C₂ à C₄, ou un groupe alkyle en C₁ à C₄ ou alkoxy en C₁ à C₄ dont chacun est substitué par du fluor ou du chlore,
n représente l'un des nombres 0, 1 et 2,
A représente l'hydrogène, le fluor, le chlore ou le brome, un reste éventuellement substitué par du fluor, du chlore ou du brome, de la série alkyle en C₁ à C₈, cycloalkyle en C₃ à C₈, alcényle en C₃ à C₈ et alcynyle en C₃ à C₈, un reste éventuellement substitué par un radical fluoro, chloro, bromo, nitro, alkyle en C₁ à C₄, alkoxy en C₁ à C₄, alkylthio en C₁ à C₄, halogénalkyle en C₁ à C₄, halogénalkoxy en C₁ à C₄ ou cyano, de la série phényle, naphtyle, furannyle, thiényle, pyridyle, phényl-(alkyle en C₁ à C₄), furannyl-(alkyle en C₁ à C₄), thiényl-(alkyle en C₁ à C₄) et pyridyl-(alkyle en C₁ à C₄) ou l'un des groupes -COR³, -CO₂R³, -CN, -CONR³R⁴, -SO₂R³ ou -P(O) (OR³)OR⁴, où
R³ et R⁴ représentent, indépendamment l'un de l'autre, l'hydrogène, un groupe alkyle en C₁ à C₈ ou alcényle en C₃ à C₈ substitué par du fluor, du chlore ou du brome ou un reste, éventuellement substitué par un radical fluoro, chloro, bromo, nitro, alkyle en C₁ à C₄, alkoxy en C₁ à C₄, alkylthio en C₁ à C₄, halogénalkyle en C₁ à C₄, halogénalkoxy en C₁ à C₄ ou cyano, de la série phényle, furannyle, thiényle, pyridyle, phényl-(alkyle en C₁ à C₄), furannyl-(alkyle en C₁ à C₄), thiényl-(alkyle en C₁ à C₄) et pyridyl-(alkyle en C₁ à C₄) , ou bien
R³ et R⁴ forment ensemble un groupe alkylène en C₂ à C₆ dans lequel, le cas échéant, un chaînon méthylène non terminal est remplacé par de l'oxygène, du soufre, un groupe NH ou N-(alkyle en C₁ à C₄),
D représente l'hydrogène, un groupe alkyle en C₁ à C₁₀, alcényle en C₃ à C₆, alcynyle en C₃ à C₆, (alkoxy en C₁ à C₈)-(alkyle en C₂ à C₆), poly-(alkoxy en C₁ à C₆)-(àlkyle en C₂ à C₆) ou (alkylthio en C₁ à C₈)-(alkyle en C₂ à C₆) dont chacun est éventuellement substitué par du fluor ou du chlore, un groupe alkyle en C₁ à C₈ éventuellement substitué par un radical cyano, (alkoxy en C₁ à C₆)-carbonyle ou (alkyle en C₁ à C₆)-carbonyloxy, un groupe cycloalkyle en C₃ à C₇ portant éventuellement un substituant fluoro, chloro, alkyle en C₁ à C₄, alkoxy en C₁ à C₄ ou halogénalkyle en C₁ ou C₂, dans lequel le cas échéant un ou deux chaînons méthylène non contigus sont remplacés par de l'oxygène et/ou du soufre, ou un groupe phényle, furannyle, imidazolyle, pyridyle, thiazolyle, pyrazolyle, pyrimidyle, pyridazyle, pyrazinyle, pyrrolyle, thiényle, triazolyle ou phényl-(alkyle en C₁ à C₄) dont chacun porte éventuellement un substituant fluoro, chloro, bromo, alkyle en C₁ à C₄), halogénalkyle en C₁ à C₄, alkoxy en C₁ à C₄, halogénalkoxy en C₁ à C₄, cyano ou nitro, ou bien
A et D forment ensemble un groupe alkylène en C₂ à C₆ ou alcénylène en C₂ à C₆ éventuellement substitué le cas échéant par un radical alkyle en C₁ à C₄, fluoro, chloro ou bromo, dans lequel le cas échéant deux atomes de carbone sont liés par un groupe alkylène en C₁ ou C₂ et dans lequel, le cas échéant, un chaînon méthylène est remplacé par de l'oxygène, du soufre, un radical NH, N-(alkyle en C₁ à C₄) ou le groupe -O-CO-,
R¹ représente l'hydrogène ou un groupe alkyle en C₁ à C₄ éventuellement substitué par du fluor, du chlore ou du brome et
R² représente un groupe alkyle en C₁ à C₈, alcényle en C₃ à C₈ ou alcynyle en C₃ à C₈ dont chacun est éventuellement substitué par du fluor, du chlore ou du brome.

4. Composés de formule (I) suivant la revendication 1, dans laquelle
X représente le fluor, le chlore, le brome, un groupe nitro, cyano, méthyle, éthyle, n-propyle, isopropyle, n-butyle, sec.-butyle, isobutyle, tertiobutyle, vinyle, allyle, méthallyle, méthoxy, éthoxy, n-propoxy, isopropoxy, allyloxy, méthallyloxy, trifluorométhyle, difluorométhoxy, trifluorométhoxy, trifluoréthoxy, méthylthio, méthylsulfinyle ou méthylsulfonyle,
Y représente l'hydrogène, le fluor, le chlore, le brome, un groupe nitro, cyano, méthyle, éthyle, n-propyle, isopropyle, n-butyle, sec.-butyle, isobutyle, tertiobutyle, méthoxy, éthoxy, n-propoxy, isopropoxy, allyloxy, méthallyloxy, trifluorométhyle, difluorométhoxy, trifluorométhoxy, trifluoréthoxy, méthylthio, méthylsulfinyle ou méthylsulfonyle,
Z représente le fluor, le chlore, le brome, un groupe nitro, cyano, méthyle, éthyle, n-propyle, isopropyle, n-butyle, sec.-butyle, isobutyle, tertiobutyle, méthoxy, éthoxy, n-propoxy, isopropoxy, allyloxy, méthallyloxy, trifluorométhyle, difluorométhoxy, trifluorométhoxy, ou trifluoréthoxy,
n représente le nombre 0 ou le nombre 1,
A représente l'hydrogène, le fluor, le chlore ou le brome, un reste, éventuellement substitué par du fluor ou du chlore, de la série alkyle en C₁ à C₆, cycloalkyle en C₃ à C₆, alcényle en C₃ à C₆ et alcynyle en C₃ à C₆, un groupe phényle, furannyle, pyridyle, thiényle ou benzyle dont chacun est éventuellement substitué par du fluor, du chlore, du brome, un radical méthyle, éthyle, n-propyle, isopropyle, méthoxy, éthoxy, trifluorométhyle, trifluorométhoxy, cyano ou nitro, ou l'un des groupes -COR³, -CO₂R³, -CN, -CONR³R⁴, -SO₂R³ ou -P(O)(OR³)OR⁴, où
R³ et R⁴ représentent, indépendamment l'un de l'autre, l'hydrogène, un groupe alkyle en C₁ à C₆ ou alcényle en C₃ à C₆ dont chacun est éventuellement substitué par du fluor ou du chlore, ou un groupe phényle portant éventuellement un substituant fluoro, chloro, bromo, nitro, alkyle en C₁ à C₄, alkoxy en C₁ à C₄, alkylthio en C₁ à C₄, halogénalkyle en C₁ à C₄, halogénalkoxy en C₁ à C₄ ou cyano, ou bien
R³ et R⁴ forment ensemble l'un des groupes -(CH₂)₃-, - (CH₂)₄-, - (CH₂)₅-, -CH₂-CH(CH₃) (CH₂)₂-, -CH₂O-(CH₂)₂- et -CH₂S-(CH₂)₂-,
D représente l'hydrogène, un groupe alkyle en C₁ à C₈, alcényle en C₃ ou C₄, alcynyle en C₃ ou C₄, (alkoxy en C₁ à C₆) - (alkyle en C₂ à C₄), poly- (alkoxy en C₁ à C₄)-(alkyle en C₂ à C₄) ou (alkylthio en C₁ à C₄) - (alkyle en C₂ à C₄) dont chacun est éventuellement substitué par du fluor ou du chlore, ou un groupe cycloalkyle en C₃ à C₆ éventuellement substitué par un radical fluoro, chloro, méthyle ou éthyle, dans lequel le cas échéant un ou deux chaînons méthylène non contigus sont remplacés par de l'oxygène et/ou du soufre, ou un groupe phényle, furannyle, pyridyle, thiényle ou benzyle portant chacun le cas échéant un substituant fluoro, chloro, bromo, méthyle, éthyle, n-propyle, isopropyle, méthoxy, éthoxy, trifluorométhyle, trifluorométhoxy, cyano ou nitro, ou bien
A et D forment conjointement l'un des groupes-(CH₂)₃-, -(CH₂)₄-, - (CH₂)₅-, -CH₂-CH(CH₃) (CH₂)₂-, -CH₂O- (CH₂)₂, -CH₂S-(CH₂)₂- ou 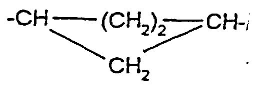
R¹ représente l'hydrogène ou un groupe méthyle ou éthyle éventuellement substitué par du fluor ou du chlore, et
R² représente un groupe méthyle, éthyle, n-propyle, isopropyle, n-butyle, isobutyle, sec.-butyle, tertiobutyle, allyle, méthallyle, butényle, propargyle ou butynyle dont chacun est éventuellement substitué par du fluor ou du chlore.

5. Procédé de production de composés de formule (I) suivant la revendication 1, **caractérisé en ce qu'**on fait réagir des composés de formule (II) 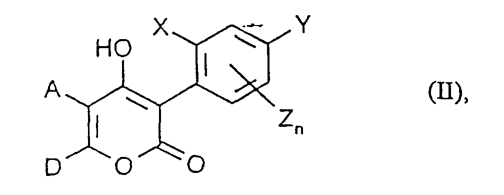 dans laquelle
A, D, X, Y, Z et n ont les définitions indiquées dans la revendication 1,
avec des composés de formule (III) 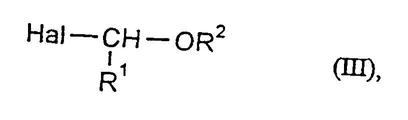 dans laquelle
R¹ et R² ont les définitions indiquées dans la revendication 1 et Hal représente un halogène,
le cas échéant en présence d'un diluant et en présence éventuelle d'un auxiliaire de réaction.

6. Compositions pesticides, **caractérisées par** une teneur en au moins un composé de formule (I) suivant la revendication 1.

7. Utilisation de composés de formule (I) suivant la revendication 1 pour combattre des parasites.

8. Procédé pour combattre des parasites, **caractérisé en ce qu'**on fait agir des composés de formule (I) suivant la revendication 1 sur les parasites et/ou sur leur milieu.

9. Procédé de préparation de compositions pesticides, **caractérisé en ce qu'**on mélange des composés de formule (I) suivant la revendication 1 avec des diluants et/ou des agents tensio-actifs.

10. Utilisation de composés de formule (I) suivant la revendication 1 pour la préparation de compositions pesticides.
